# EUROPEAN PATENT SPECIFICATION

(11) **EP 2 187 213 B1**
(45) Date of publication and mention of the grant of the patent: **24.04.2013**
(21) Application number: 09168598.2
(22) Date of filing: 25.08.2009
(51) Int. Cl.: G01N 33/542, G01N 33/557, G01N 33/58, G01N 33/68, G01N 33/50, C12N 15/10

(54) **Methods for detecting interactions between two and more polypeptides**
Verfahren zur Erkennung von Wechselwirkungen zwischen zwei oder mehr Polypeptiden
Procédés de détection d'interactions entre deux polypeptides ou plus

(30) Priority: 12.11.2008 KR 20080112354
(43) Date of publication of application: 19.05.2010
(73) Proprietor: Korea Basic Science Institute, Yuseong-gu 305-333 Daejeon (KR)
(72) Inventor: Lee, Zee Won, 305-755 Daejeon (KR); Kim, Soo Hyun, 305-755 Daejeon (KR)
(74) Representative: ABG Patentes, S.L.

(56) References cited:
- WO-A1-00/17221
- WO-A1-2009/049892
- WO-A2-2008/060483
- KNAUER S K ET AL: "Translocation biosensors to study signal-specific nucleo-cytoplasmic transport, protease activity and protein-protein interactions." TRAFFIC, vol. 6, no. 7, July 2005 (2005-07), pages 594-606, XP002551154
- TANAKA M ET AL: "Yellow fluorescent protein-tagged and cyan fluorescent protein-tagged imaging analysis of glucocorticoid receptor and importins in single living cells." ENDOCRINOLOGY, vol. 144, no. 9, September 2003 (2003-09), pages 4070-4079, XP002551155
- Taketoshi Kajimoto ET AL: "Ceramide-induced Apoptosis by Translocation, Phosphorylation, and Activation of Protein Kinase C in the Golgi Complex", Journal of Biological Chemistry, vol. 279, no. 13, 1 March 2004 (2004-03-01), pages 12668-12676, XP55001833, ISSN: 0021-9258, DOI: 10.1074/jbc.M312350200

## Description

### FIELD OF THE INVENTION

Disclosed is a novel method for detecting interactions of biomolecules. More particularly, the disclosed method includes (a) preparing a cell comprising (i) a first construct comprising a bait, a first labeling material and a translocation module; and (ii) a second construct comprising a prey and a second labeling material; (b) detecting the distribution of the first construct and the second construct in the cell.

### BACKGROUND OF THE INVENTION

Growth, differentiation, migration, death and the like of cells are mediated by macromolecular interactions such as protein-protein or protein-nucleic acid interactions. Signals from outside of cells pass through receptors located on the cellular membrane and are transmitted to the nucleus of a cell through various biochemical reactions, where they express specific genes. This transfer of external signals into a cell is accomplished by protein interactions of several stages. For example, growth factors or cytokines bind to corresponding cell-surface receptors. This binding induces the receptors to cluster. The clustering of receptors by ligands induces clustering of the intracellular domains of the receptors, thereby causing interactions with signaling-related proteins. Through this signaling mechanism, intermediate proteins capable of transferring signals are produced by phosphorylation by protein kinases, dephosphorylation by protein phosphatases, or the like. As a result, the signals are transmitted to transcriptional activator proteins (Helden, C. H., (1995) Cell 80, 213-223). The activated transcriptional activators bind to DNAs and interact with basal transcriptional regulator proteins such as RNA polymerases to activate specific genes. Such interactions enable transcription to occur specifically in specific tissues during embryologic processes or in response to external stimulations. Abnormal modification, inhibition or acceleration of such interactions between specific proteins, which may caused by intrusion of foreign matters, genetic modification of internal activator proteins, or the like, may be the cause of a disorder. Accordingly, there have been consistent researches because substances that can regulate the interactions may provide a way to treat the disorder.

The methods for analyzing the interactions of biomolecules, particularly the binding properties thereof, include traditional in vitro methods such as cross-linking, affinity chromatography, immunoprecipitation (IP), or the like. These methods require the production, isolation and purification of protein and are disadvantageous in that an information different from the actual interaction may be obtained depending on the buffer condition in the test tube, the secondary modification of extracted proteins, or the like.

In order to make up for these drawbacks of the in vitro methods, in-cell methods such as yeast two-hybrid (Y2H), fluorescence resonance energy transfer (FRET) and bimolecular fluorescence complementation (Bi-FC) techniques have been developed. These methods have advantages and disadvantages mentioned below.

Y2H is currently the most widely used technique along with immunoprecipitation. It is advantageous in that large-scale screening is possible using a gene library, but is disadvantageous in that investigation of membrane proteins or nuclear proteins such as transcriptase is difficult and there is a high probability of false positive. Besides, this method is inappropriate to find a substance capable of regulating protein-protein interactions. In the Y2H technique, the interaction between two proteins is detected based on the color change of colony to blue as X-gal is decomposed when β-galactosidase is expressed by the reporter gene. Since the screening technique of detecting the color change from blue back to white by a candidate substance is a negative screening, it is probable that a substance which has actually an inhibitory effect may be unnoticed. Further, since the detection itself is somewhat ambiguous, the technique is not suitable for general drug screening.

The FRET method provides good accuracy, but it is disadvantageous in that positioning of fluorescent proteins or fluorescent materials, which is required for the fluorescence resonance energy transfer to occur, is difficult, thereby having low rate of experimental success. The Bi-FC method is advantageous in that it is applicable to membrane proteins or nuclear proteins. However, like the FRET method, it is disadvantageous in that relative positioning of proteins for complementary binding is difficult, thereby having low rate of success.

Therefore, various modified methods have been proposed to overcome the disadvantages of the above-described methods.

WO 2008/060483 A2 discloses methods and reagents for identifying an agent that modulates the interaction of two or more polypeptides, the method comprising: introducing into a cell at least a first polypeptide, each comprising a binding domain, wherein the first polypeptide comprises a localization domain of the second polypeptide; and detecting the cellular location of the first polypeptide, the second polypeptide or a combination thereof, wherein a change in the cellular location of the first polypeptide, the second polypeptide or a combination thereof indicates that the agent modulates the interaction of the two or more polypeptides.

Knauer et al. (Traffic. 2005 Jul;6(7):594-606) teaches Translocation biosensors to study signal-specific nucleo-cytoplasmic transport, protease activity and protein-protein interactions.

Tanaka et al. (Endocrinology. 2003 Sep;144(9):4070-9) relates to yellow fluorescent protein-tagged and cyan fluorescent protein-tagged imaging analysis of glucocorticoid receptor and importins in single living cells.

WO 2000/017221 A1 discloses a method of detecting protein-protein interactions in a living cell comprises (a) providing a cell that contains a first heterologous conjugate and a second heterologous conjugate, wherein the first heterologous conjugate comprises a first protein of interest conjugated to a detectable group, and wherein the second heterologous conjugate comprises a second protein of interest conjugated to a protein that specifically binds to an internal structure within the cell, and then (b) detecting the presence or absence of binding of the detectable group to the internal structure, the presence of the binding indicating that the first and second proteins of interest specifically bind to one another.

WO/2009/049892 relates to a first polypeptide (A) comprising a recruiting polypeptide (a) comprising at least an annexin core domain or a functional variant thereof, a bait polypeptide (b) and a luminophore. It also provides a composition comprising at least one first polypeptide (A) and further comprising at least a second polypeptide (B) comprising, a first target polypeptide (d) and a luminophore (e).

Kajimoto et al. (J Biol Chem. 2004 Mar 26;279(13):12668-76) relates to Ceramide-induced apoptosis by translocation, phosphorylation, and activation of protein kinase Cdelta in the Golgi complex.

However, there is a consistent need for an effective method for detecting the binding of biomaterials. Particularly, a detecting system enabling the detection of proteins interacting with target proteins and enabling a more efficient detection of regulator materials that inhibit or promote the interactions between two proteins is urgently needed.

### SUMMARY OF THE INVENTION

The inventors of the present invention have researched to develop a method enabling the real-time detection of binding and interactions of materials in living cells. As a result, we have found that the interaction of a bait and a prey in a living cell can be detected in real time by using a first construct which includes a translocation module moving to a specific region in a cell in response to an external signal or via an internal signaling mechanism and a bait which is a target of an interaction, and a second construct which includes a prey which is another target of the interaction. The first construct and the second construct are labeled with a labeling material, so that the interaction of the bait and the prey in a living cell can be detected in real time by tracing their movements in the cell.

Accordingly, the present invention provides a novel method enabling the detection of bait and a prey which interact with each other.

To achieve the above object, the present invention provides the method for detecting interactions between bait and prey comprising the steps of:
(a) preparing a cell comprising (i) a first construct comprising a bait, a first labeling material and a translocation module; and (ii) a second construct comprising a prey and a second labeling material; and
(b) detecting the distribution of the first construct and the second construct in the cell.

To achieve another object, the present invention provides the screening method for materials changing interaction between bait and prey comprising the steps of:
(a) preparing a cell comprising (i) a first construct comprising a bait, a first labeling material and a translocation module; and (ii) a second construct comprising a prey and a second labeling material;
(b) treating with a signaling material; and
(c) detecting the distribution of the first construct and the second construct in the cell.

To achieve another object, the present invention provides the cells comprising (i) a first construct comprising bait, a first labeling material and a translocation module; and (ii) a second construct comprising a prey and a second labeling material.

To achieve another object, the present invention provides the kit for detecting interactions between bait and prey comprising the said cells.

To achieve another object, the present invention provides the use of the kit of claim 14 for detecting interactions of a bait and a prey and/or for screening materials which alter interactions of a bait and a prey

### BRIEF DESCRIPTION OF THE DRAWINGS

The above and other aspects, features and advantages of the disclosed exemplary embodiments will be more apparent from the following detailed description taken in conjunction with the accompanying drawings in which:
FIG. 1 illustrates basic constructs and analysis indices of the present invention;
FIG. 2 illustrates a basic concept of binding and non-binding in accordance with the present invention;
FIG. 3 schematically illustrates the analysis of binding and non-binding in accordance with the present invention;
FIG. 4 shows an example of first construct (A) and second construct (B) in accordance with the present invention;
FIG. 5 shows the change of the distribution of the first construct and second construct in response to an external stimulation;
FIG. 6 shows the change of the distribution of the first construct (pTMD-mRFP-C3 vector) depending on the PMA concentration;
FIG. 7 shows translocation efficiency and distribution of first construct (TMD-mRFP-Bait, TMA-mRFP-Bait and TMB-mRFP-Bait);
FIG. 8 shows distribution of first construct (TMD-mRFP-p53-NES) and second construct;
FIG. 9 shows distribution of first construct (TMD-mRFP-p53-NLS) and second construct;
FIG. 10 shows the result of analyzing the binding of p53 protein and SV40T protein using the method of the present invention;
FIG. 11 shows the result of analyzing the binding of lysyl-tRNA synthetase (KRS) and JTV1 (p38), Gag and laminin receptor (LR);
FIG. 12 shows the result of analyzing the binding of RelA and IkB in real time using a confocal laser fluorescence microscope;
FIG. 13 shows the result of analyzing the binding of three protein complexes;
FIG. 14 shows the result of verifying the binding of positive candidates screened through Y2H;
FIG. 15 shows the result of analyzing the binding of p53 protein with mdm2 protein and the inhibition of binding by the anticancer drug nutlin3; and
FIG. 16 shows the result of analyzing the binding inhibition concentration of the anticancer drug nutlin3.

### DETAILED DESCRIPTION OF THE INVENTION

Hereafter, the present invention will be described in detail.

In the present invention, translocation of proteins in a cell in response to an external signal or via an internal signaling mechanism is monitored to detect the interact ion between biomaterials in a cell directly and in real time. A first construct is designed by fusing a bait, which is a target of an interaction, with a translocation module, which relocates in response to an external signal or via an intrinsic signaling mechanism, and with a labeling material to trace it. Further, a second construct is designed to comprise a prey interacting with the bait and another labeling material enabling tracing thereof. The first construct and the second construct are made to exist in a cell at the same time. As a result, the interaction between them in a cell can be analyzed directly in real time.

Accordingly, the present invention provides the method for detecting interactions between bait and prey comprising the steps of:
(a) preparing a cell comprising (i) a first construct comprising a bait, a first labeling material and a translocation module; and (ii) a second construct comprising a prey and a second labeling material; and
(b) detecting the distribution of the first construct and the second construct in the cell.

As used herein, the bait (i.e., the molecule of interest) and the prey (i.e., the target molecule) refer to the materials that are subject to an interaction. Each of the bait and the prey is a polypeptide . For the purpose of detection or screening of an interaction, the bait may be a known material and the prey may be an unknown material. But, without being limited thereto, the bait and the prey may be interchangeably included in the first construct or second construct.

As used herein, a first labeling material and a second labeling material refer to fluorescent proteins capable of generating a signal that can be detected by those skilled in the art. Those fluorescent proteins which are well known in the art may be used. Examples may include GFP (Green Fluorescent Protein); EGFP (Enhanced Green Fluorescent Protein); RFP (Red Fluorescent Protein); mRFP (Monomeric Red Fluorescent Protein); DsRed (Discosoma sp. red fluorescent protein); CFP (Cyan Fluorescent Protein); CGFP (Cyan Green Fluorescent Protein); YFP (Yellow Fluorescent Protein); AzG (Azami Green), HcR (HcRed, Heteractis crispa red fluorescent protein), BFP (Blue Fluorescent Protein).

A first labeling material and a second labeling material of the present invention are fluorescent proteins. Preferably, a first labeling material and a second labeling material of the present invention may be GFP; EGFP; RFP; mRFP; DsRed (Discosoma sp. red fluorescent protein); CFP (Cyan Fluorescent Protein); CGFP (Cyan Green Fluorescent Protein); YFP (Yellow Fluorescent Protein); AzG (Azami Green), HcR (HcRed, Heteractis crispa red fluorescent protein), or BFP (Blue Fluorescent Protein). According to the invention, a first labeling material and a second labeling material are different for distinction. Preferably, a first labeling material and a second labeling material of the present invention may have an amino acid sequence represented by SEQ ID NO:9 (EGFP) SEQ ID NO:11 (mRFP), SEQ ID NO:13 (AzG) or SEQ ID NO:15 (HcR) or a nucleotide sequence represented by SEQ ID NO:10 (EGFP), SEQ ID NO:12 (mRFP), SEQ ID NO:14 (AzG) or SEQ ID NO:16 (HcR).

In the present invention, the translocation module serves to move the first construct to a specific region in a cell. The translocation to the specific region may be induced by an external signal or induced intrinsically. The specific region in a cell refers to an intracellular structure which is separate, discreet and identifiable. Preferably, the specific region may be membranous (structures such as cell membrane, nuclear membrane, etc.), organelles such as endoplasmic reticulum, Golgi apparatus, mitochondria, lysosome, etc., or other specific regions in a cell.

The translocation module of the present invention has an amino acid sequence represented by SEQ ID NO: 7 (TMD), or a nucleotide sequence represented by SEQ ID NO: 8 (TMD).

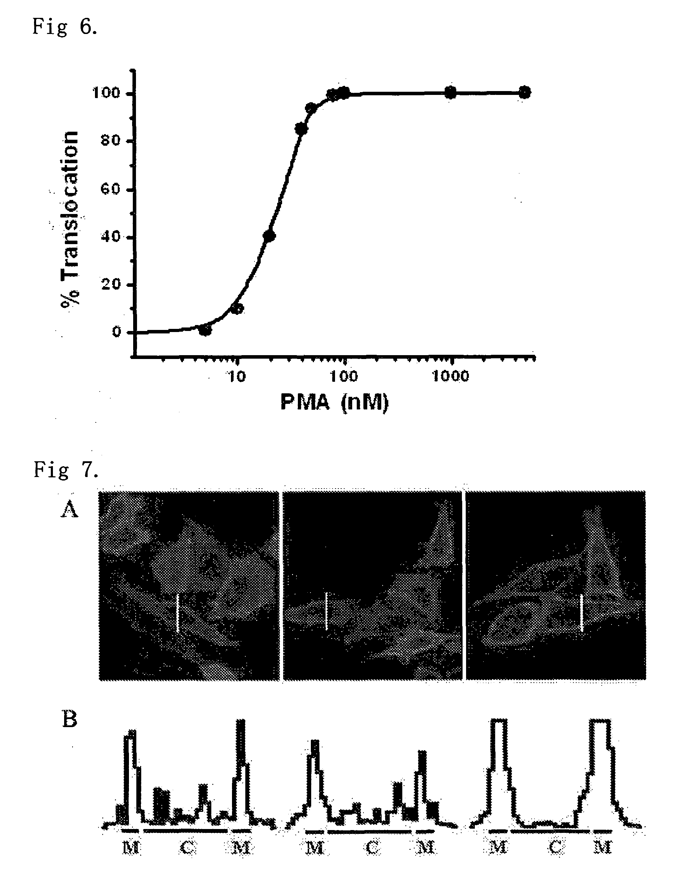

From the analysis of translocation efficiency of several proteins including RasGRP, which migrates by signals, and C1 domains through preliminary experiments, it was confirmed that the mutant denoted as TMD of the present invention exhibited relatively superior translocation efficiency (see FIG. 7).

The first construct or second construct of the present invention may further include a nuclear localization signal (NLS) or nuclear export signal (NES). These may be further included to control the change of intracellular distribution depending on the intrinsic properties of the bait or prey, or on the particular cell line used in the experiment. In case an NLS is further added, it may direct the first construct and second construct to be distributed uniformly in the nucleus. And, if an NES is further added, it may direct the first construct and second construct to be distributed uniformly in the cytoplasm. Through this, it is possible to recognize whether the binding of the bait and the prey occurs in the cytoplasm or nucleus, or to induce the binding occur in the cytoplasm or nucleus. NLS may preferably have a sequence which is well known in the art [for example, SV40 T Antigen (PKKKRKV) (SEQ ID NO:93), Yeast histone H2B (GKKRSKV) (SEQ ID NO:94), Human c-myc (PAAKRVKLD) (SEQ ID NO:95), Nucleoplasmin (KRPAATKKAGQAKKKKL) (SEQ ID NO:96), Human IL-5 (KKYTDGQKKKCGEERRRVNQ) (SEQ ID NO:97), Human RB (KRSAEGSNPPKPLKKLR) (SEQ I D NO:98), Human p53 (KRALPNNTSSSPQPKKKP) (SEQ ID NO:99)] or the amino acid sequence represented SEQ ID NO: 17, more preferably it may have the amino acid sequence represented SEQ ID NO: 17 (GSGDEVEGVEEVAKKKSKKEKDK) or the nucleotide sequence represented by SEQ ID NO:18 which encode thereof (ggctctggtgatgaagtcgaaggagtggaagaagtagctaagaagaagagtaaaaaggaaaaggataaa). In addition, NES may have a sequence which is well known in the art [for example, Annexin II (VHEILCK--LSLE) (SEQ ID NO:78), mNet (TLWQF-LLH--LLLD) (SEQ ID NO:79), hNet (TLWQF-LLQ--LLLD) (SEQ ID NO:80), MAPKK (ALQKK-LEE--LELD) (SEQ ID NO:81), PKI (ELALK-LAG--LDIN) (SEQ ID NO:82), Rev (LQLPPLER--LTLD) (SEQ ID NO:83), Dsk-1 (SLEGAVSEIS-LR) (SEQ ID NO:84), Cyclin B1 (YLCQAFSDVI-LA) (SEQ ID NO:85), ANXII (STVHEILCK--LSLE) (SEQ ID NO:86), HIV-1 Rev (LQLPPLER--LTLD) (SEQ ID NO:87), MEK-1 (ALQKK-LEE--LELD) (SEQ ID NO: 88), PKI-α (ELALK-LAG--LDIN) (SEQ ID NO:89), IkB-α (IQQQLGQ--LTLE) (SEQ ID NO:90), RanBP1 (KBAEKLEA--LSVR), INI1 (DQRVIIKLNAHVGNISLV) (SEQ ID NO:91)] or amino acid sequence represented SEQ ID NO 19, more preferably it may have the amino acid sequence represented SEQ ID NO: 19 (DQRVIIKLNAHVGNISLV) or the nucleotide sequence represented by SEQ ID NO:20 (gaccagcgcgtcatcatcaagctgaacgcccatgtgggaaacatttccctggtg) which encode thereof.

The detection of the distribution of the first construct and second construct in a cell is carried out using a labeling material in accordance with a detection method commonly known in the art. For example, a fluorescence microscope may be used to detect the distribution of the first construct and second construct in a cell.

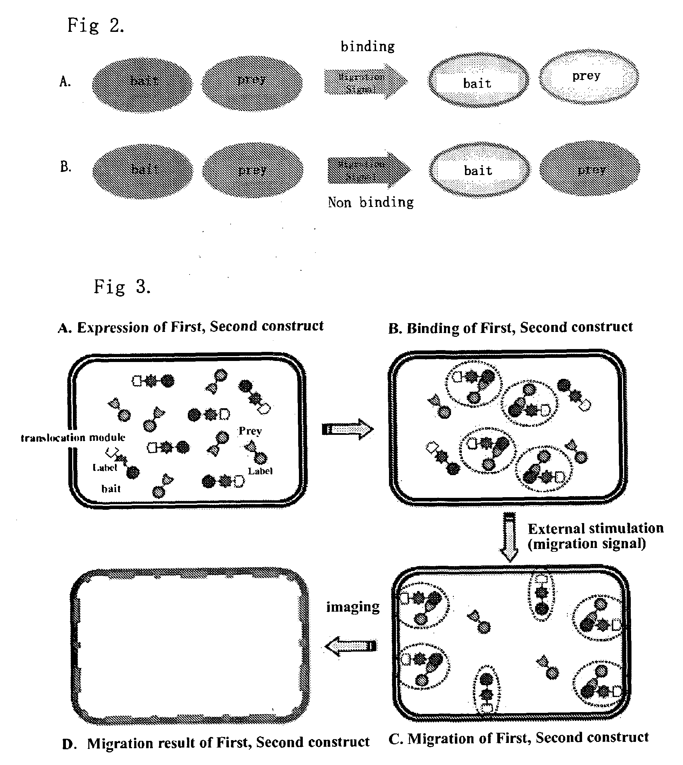

Initially, both the first construct and second construct are randomly distributed in the cytoplasm or nucleus (a translocation module moving via an intrinsic signaling mechanism moves to a specific region). When receiving a translocation signal, the first construct is moved toward the cell membrane by the translocation module. At this time, the prey bound to the bait is also carried toward the cell membrane. In contrast, unless the prey is not bound to the bait, its distribution will not change. Accordingly, the binding of the bait and the prey can be recognized by the translocation of the prey toward the cell membrane (see FIGS. 2 and 3).

Thus, the method of the present invention enables real-time monitoring of direct binding or complex of biomolecules in a living cell through imaging, and provides the following advantages over existing techniques.
1) All bindings occurring in a living cell can be analyzed.
2) Analysis in tissue or individual level is possible.
3) Applicable to animal cells, yeast and bacteria.
4) No antibodies for the bait and prey are required.
5) Accurate analysis is possible because the positional change in a cell is monitored, differently from other methods where the whole cell is monitored.
6) 3-dimensional analysis is unnecessary because 2-dimensional positional change is analyzed.
7) It is not necessary to use the expensive confocal microscope because 3-dimensional analysis is unnecessary.
8) Binding analysis is possible for various cell organelles.
9) Not influenced by external environment as in the in vitro method, because binding occurring in a living cell is monitored.
10) The binding of a bait and a prey can be monitored in real time.
11) The binding of a bait with multiple preys can be monitored.
12) False positive can be minimized through all-or-none monitoring.
13) Interfering bindings due to inflow of extracellular material can be excluded ultimately by using only genes of a bait and a prey and stimulating materials.
14) Complementary screening of protein binding is possible through various modifications of the translocation module.
15) Detection of permanent binding, transient binding and instantaneously occurring interaction is possible.
16) Targets of currently developed signaling inhibitors can be specified.
17) Re-evaluation of inhibitors (drug repositioning/repurposing) is possible through target specification of inhibitor.
18) Screening of binding of a prey to an unknown biomaterial is possible using a mass marker library for the prey.
19) A high-throughput system can be implemented in association with a high-content screening (HCS) system.
20) Simple analysis is possible based on stimulation-free movement.
21) Binding properties can be analyzed for different signaling pathways by changing external stimulation.
22) Relative quantitation of the bait and prey is possible by labeling both the first construct and second construct with a labeling material.
23) False positive or false negative responses can be significantly reduced because the experimental errors related to the translocation of the prey in response to external stimulation or via intrinsic signaling mechanism can be simultaneously verified.

As described, the translocation module may move to a specific region in a cell in response to an external signal. Accordingly, the detection of an interaction between a bait and a prey in accordance with the present invention may further comprise treating with a signaling material. That is, the method may comprise:
(a) preparing a cell comprising (i) a first construct comprising a bait, a first labeling material and a translocation module; and (ii) a second construct comprising a prey and a second labeling material;
(b) treating with a signaling material; and
(c) detecting the distribution of the first construct and the second construct in the cell.

The signaling material refers to a material which generates an external signal inducing the translocation of the translocation module. For example, the signaling material may be phorbol-12-myristate 13-acetate (PMA),12-O-tetradecanoylphorbol-13-acetate (TPA), phorbol-12,13-dibutyrate (PDBu), adenosine triphosphate (ATP), tridecanoic acid, arachidonic acid, linoleic acid, DiC8, 130C937, PKC activation-related growth factors or other PKC activating materials.

PMA may be treated at a concentration of preferably 50 nM to 5 µM, more preferably 1 µM. If the PMA concentration is below 50 nM, translocation of the PKC translocation module may be insufficient. Otherwise, if it exceeds 5 µM, excessive treatment of the chemical may result in undesired phenomena as cell death, signaling interference, or the like.

Also, the present invention provides the method for screening materials which alter interactions of bait and prey comprising the steps of:
(a) preparing a cell including (i) a first construct including a bait, a first labeling material and a translocation module; and (ii) a second construct including a prey and a second labeling material;
(b) treating with a test agent; and
(c) detecting the distribution of the first construct and the second construct in the cell.

As used herein, the term "test agent" includes any substance, molecule, element, compound, entity or a combination thereof. For example, it includes protein, polypeptide, small organic molecule, polysaccharide, polynucleotide, or the like, but is not limited thereto. Further, it may be a natural product, synthetic compound or chemical compound, or a combination of two or more of them. Unless specified otherwise, the terms agent, substance, material and compound may be used interchangeably.

The test agent screened or identified by the method of the present invention may comprise polypeptide, beta-turn mimetics, polysaccharide, phospholipid, hormone, prostaglandin, steroid, aromatic compound, heterocyclic compound, benzodiazepines, oligomeric N-substituted glycines, oligocarbamates, saccharides, fatty acid, purine, pyrimidne or derivatives thereof, structural analogues or mixtures thereof. The said test agent may be driven from broad and various origins which comprise artificial synthesis or library of natural compounds. Preferably, the said test agent may be peptide of, for example, about 5-30, preferably 5-20, more preferably 7-15 amino acids. The said peptide may be naturally generated protein, random peptide, or fragment of "biased" random peptide.

In addition, the said test agent may be "nucleic acid". The nucleic acid test agent may be naturally generated nucleic acid, random nucleic acid, or "biased" random nucleic acid. For example, the fragment of prokaryotic genome or eukaryotic genome may be used as described above.

Further, the test agent may be a small compound molecule (e.g., a molecule having a molecular weight of about 1,000 or smaller). Preferably, high throughput assay may be employed to screen a small molecular agent.

The change of the interaction between the bait and the prey may be either an inhibition or enhancement of the interaction. The inhibition of the interaction refers to the inhibit ion of the binding between the bait and the prey. In the method of the present invention, the inhibition of the interaction, for example, may be determined from the absence (or decrease of frequency, degree or extent) of translocation of the second construct when the test agent is treated, as compared to that comparable to the translocation of the first construct when the test agent is not treated. The enhancement of the interaction refers to the enhancement of the binding between the bait and the prey. In the method of the present invention, the enhancement of the interaction, for example, may be determined from the presence (or increase of frequency, degree or extent) of translocation of the second construct comparable to that of the first construct when the test agent is treated, as compared to the absence of translocation of the second construct when the test agent is not treated.

In addition, the said screening method may further comprise the step of treating with a signaling material. For example, it may be the method comprising the steps of:
(a) preparing a cell including (i) a first construct including a bait, a first labeling material and a translocation module; and (ii) a second construct including a prey and a second labeling material;
(b) treating with a signaling material;
(c) treating with a test reagent; and
(d) detecting the distribution of the first construct and the second construct in the cell.

However, the step of treating with a signaling material does not need to be performed prior to the step of treating with a test reagent, and the skilled in the art may control the procedures.

In addition, the present invention provides a cell including (i) a first construct including a bait, a first labeling material and a translocation module; and (ii) a second construct including a prey and a second labeling material.

The cell may be a cell of an animal, plant, yeast or bacteria. Preferably, except for bacteria, it may be a cell capable of accepting the first construct introduced from outside well and having well-defined boundaries of cytoplasm, nucleus and organelles. More preferably, the cell may be CHO-k1 (ATCC CCL-61, *Cricetulus griseus,* hamster, Chinese), HEK293 (ATCC CRL-1573, *Homo sapiens,* human), HeLa (ATCC CCL-2, *Homo sapiens,* human), SH-SY5Y (ATCC CRL-2266, *Homo sapiens,* human), Swiss 3T3 (ATCC CCL-92, *Mus musculus,* mouse), 3T3-L1 (ATCC CL-173, *Mus musculus,* mouse), NIH/3T3 (ATCC CRL-1658, *Mus musculus,* mouse), L-929 (ATCC CCL-1, *Mus musculus,* mouse), Rat2 (ATCC CRL-1764, *Rattus norvegicus,* rat), RBL-2H3 (ATCC CRL-2256, *Rattus norvegicus,* rat), MDCK (ATCC CCL-34, *Canis familiaris*). In addition, the cell may be stem cells, cells extracted from tissues and the artificially made mimic cell membrane structure.

The present invention further provides a kit for detecting interaction comprising a cell comprising the first construct and the second construct of the present invention, and the use of the kit said kit for detecting interactions of a bait and a prey and/or for screening materials which alter interactions of a bait and a prey.

The kit of the present invention may further comprise a tool and/or reagent known in the art used for the detection of a labeling material, in addition to the cell comprising the first construct and second construct. The kit of the present invention may further include a tube, well plate, instruction manual, or the like, if necessary.

The experimental procedures, reagents and reaction conditions that can be used in the method of the present invention may be those commonly known in the art and will be readily understood by those skilled in the art.

In the present invention, the cell comprising the first construct and second construct may be prepared by a molecular biology technique known in the art. Although not limited thereto, expression vectors capable of expressing the first construct and the second construct, respectively, or an expression vector capable of expressing both the first construct and second construct may be introduced into a cell, so that the first construct and second construct are expressed by the expression vector(s). To this end, for the first construct, an expression vector comprising a promoter (first promoter) and a polynucleotide encoding a bait, a first labeling material and a translocation module, which is operably linked thereto, may be constructed, and, for the second construct, an expression vector comprising a promoter (second promoter) and a polynucleotide encoding a prey and a second labeling material, which is operably linked thereto, may be constructed. The two expression vectors may be simultaneously or sequentially introduced into a single cell, so that the first construct and second construct are expressed by the expression vectors. The sequence of the bait, the first labeling material and the translocation module in the polynucleotide is not important, as long as the function of the present invention is exerted. The same is true of the polynucleotide encoding the prey and the second labeling material.

The "promoter" means a DNA sequence regulating the expression of nucleic acid sequence operably linked to the promoter in a specific host cell, and the term "operably linked" means that one nucleic acid fragment is linked to other nucleic acid fragment so that the function or expression thereof is affected by the other nucleic acid fragment. Additionally, the promoter may include an operator sequence for controlling transcription, a sequence encoding a suitable mRNA ribosome-binding site, and sequences controlling the termination of transcription and translation. Additionally, it may be a constitutive promoter which constitutively induces the expression of a target gene, or inducible promoter which induces the expression of a target gene at a specific site and a specific time, and examples thereof include a SV40 promoter, CMV promoter, CAG promoter (Hitoshi Niwa et al., Gene, 108:193-199, 1991; Monahan et al., Gene Therapy, 7:24-30, 2000), CaMV 35S promoter (Odell et al., Nature 313:810-812, 1985), Rsyn7 promoter (US Patent Application No. 08/991,601), rice actin promoter (McElroy et al., Plant Cell 2:163-171, 1990), Ubiquitin promoter (Christensen et al., Plant Mol. Biol. 12:619-632, 1989), ALS promoter (US Patent Application No. 08/409,297). Also usable promoters are disclosed in US Patent No. 5,608,149; 5,608,144; 5,604,121; 5,569,597; 5,466,785; 5,399,680; 5,268,463; 5,608,142, etc.

The introduction of expression vector to a cell may be performed by the transfection methods which are well known in the art, for example, calcium phosphate method, calcium chloride method, rubidium chloride method, microprojectile bombardment, electroporation, particle gun bombardment, Silicon carbide whiskers, sonication, PEG-mediated fusion, microinjection, liposome-mediated method, magnetic nanoparticle-mediated method.

Meanwhile, general recombinant DNA and molecular cloning techniques of the present invention are well known in the art and they are well describe in the following references Sambrook, J., Fritsch, E. F. and Maniatis, T., Molecular Cloning: A Laboratory Manual, 2nd ed., Cold Spring Harbor Laboratory: Cold Spring Harbor, NY (1989); by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience (1987).

Hereinafter, the embodiments of the present invention will be described in detail with reference to accompanying drawings.

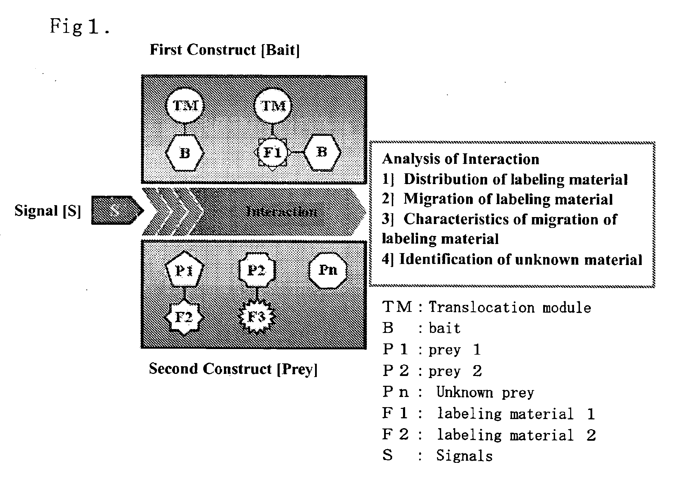

FIG. 1 illustrates basic constructs and analysis indices of the present invention. 1) A first construct comprises a translocation module, a first labeling material and a bait. The first construct may comprise a single bait or a plurality of baits. 2) A second construct comprises a second labeling material and a prey. The second construct may comprise a single prey or a plurality of baits. 3) A signal S includes an extrinsic stimulation inducing the movement of the translocation module [growth factors, serum factors, PMA, etc. that induce the positional change of translocation module], intrinsic stimulation (DAG), and cellular concentration change of ATP or calcium intrinsically or extrinsically. 4) Interaction analysis indices include the distribution of the position of the first construct and second construct by the intrinsic or extrinsic characteristics of baits or preys, change of the distribution, translocation characteristics of the constructs, identification of complexes formed by one or more of the first and second constructs, or the like.

FIG. 2 illustrates a basic concept of binding and non-binding in accordance with the present invention. The bait and prey expressed in the cell are distributed randomly in the cytoplasm or nucleus. In response to a translocation signal, the first construct is carried toward the cell membrane by the translocation module (bait, red). The prey bound to the bait is also carried toward the cell membrane (A; prey, green). In contrast, the prey not bound to the bait does not change its original distribution (B; prey, green). Accordingly, the movement of the prey toward the cell membrane reflects the binding between the bait and prey.

FIG. 3 schematically illustrates the analysis of binding and non-binding in accordance with the present invention. For example, if plasmid vectors wherein the first construct and the second construct are encoded, respectively, are introduced into a cell at the same time, the two constructs are overexpressed in the cell (see A), resulting in the binding of the bait and prey by the intrinsic interaction property. However, in this state, the binding cannot be recognized because the two fluorescent labels exist together as seen in FIG. 2 (see B). When an external stimulation (1 µM PMA) is applied in order to confirm the interaction between baits and preys, the first construct comprising the translocation module moves toward the cell membrane, and the prey bound to the bait is also carried toward the cell membrane (see C). Accordingly, the binding between the bait and prey can be detected from the co-localized translocation of the two fluorescent labels. By labeling both the first construct and second construct with a labeling material and tracing them, the experimental accuracy can be improved as compared to when only the first construct or the second construct is labeled with a labeling material. If only the first construct is labeled with a labeling material, it cannot be confirmed whether the prey binds to the bait. And, if only the second construct is labeled with a labeling material, it cannot be confirmed whether the movement of the prey is due to the binding with the bait, in case the prey has mobility in response to an external stimulation or due to an internal cause.

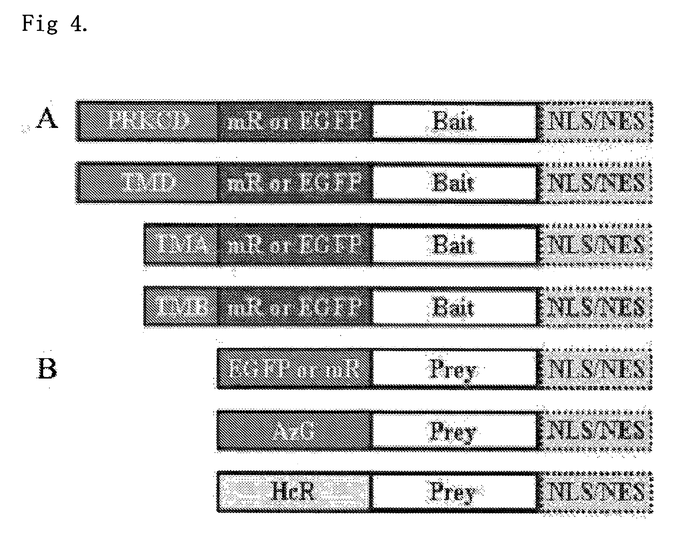

FIG. 4 shows an example of first construct (A) and second construct (B) in accordance with the present invention. Basically, the first construct comprises a translocation module (TMD), a fluorescent protein such as red fluorescent protein (mR; mRFP) or green fluorescent protein (EGFP), and a multicloning gene sequence (Bait) for the bait. If it is.needed to change the location of the bait or prey in the cell, the construct may further include a nuclear localization signal (NLS) or a nuclear export signal (NES). The second construct comprises a fluorescent protein (any fluorescent protein distinguishable from that of the first construct) for identifying the movement of the prey, and a multicloning gene sequence (Prey) for the prey. The fluorescent protein may be EGFP, mRFP, Azami Green (AzG), HcR, etc. A variety of distinguishable combinations may be attained using currently known fluorescent proteins, depending on the analysis tools (microscope, etc.) to be used.

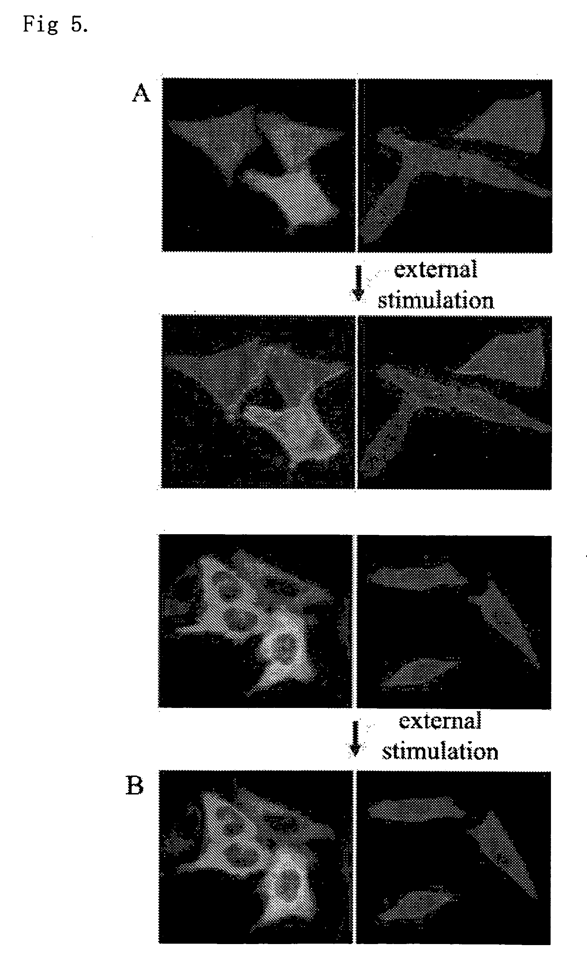

FIG. 5 shows the change of the distribution of the first construct vector and second construct vector in response to an external stimulation. Basic first constructs with no bait cloned (i.e., without a bait) [TMD-EGFP-Bait (A, left panel), TMD-mRFP-Bait (A, right panel)] and basic second constructs with no prey [EGFP-Prey (B, left panel), mRFP-Prey (B, right panel)] were overexpressed in CHO-kl cell line, and the distribution of the location of the two constructs was confirmed after applying an external stimulation (1 µM PMA). The EGFP- or mRFP-labeled first construct moved toward the cell membrane in response to the external stimulation (see A), whereas the second construct not comprising the translocation module showed no change of fluorescence distribution in response to the external stimulation (see B). Accordingly, it can be seen that the change of the distribution of the second construct is a passive phenomenon occurring due to other factor (the first construct).

FIG. 6 shows the change of the distribution of the first construct (TMD-mRFP) depending on the PMA concentration. In order to optimize the translocation characteristic of the translocation module, the first construct vector (pTMD-mRFP-C3) shown in FIG. 5 was used to determine the optimum concentration of PMA (phorbol-12-myristate-13-acetate), which was used as an external stimulation. Detailed experimental procedures were the same as in FIG. 5. After treating with PMA at concentrations of 1 nM to 5 µM, the cells in which red fluorescence moved toward the cell membrane were counted among randomly selected fluorescence-exhibiting cells. As seen in the table, IC₅₀ was measured as 35 nM. At 50 nM, 90% or more translocation was observed. At 100 nM or higher concentrations, movement of red fluorescence toward the cell membrane increased. Therefore, all experiments were carried out at 1 µM, which is a concentration sufficient for the response and movement of most cells.

FIG. 7 compares translocation efficiency of the first constructs [TMD-mRFP (right panel), TMA-mRFP (left panel), TMB-mRFP (central panel)]. In order to determine the optimum translocation module for the first construct, translocation efficiency of the first construct vector shown in FIG. 5 (pTMD-mRFP-C3), a pTMA-mRFP-C3 vector comprising a TMD fragment (a vector prepared by inserting TMA translocation module into the mRFP-C3 vector, see Example 2), and a pTMB-mRFP-C3 vector (a vector prepared by inserting TMB translocation module into the mRFP-C3 vector, see Example 2) was evaluated. Detailed experimental procedures were the same as in FIG. 5. All the three constructs exhibited change of distribution in response to an external stimulation (see A). There was no statistical difference in the number of cells with changed distribution. However, when the intensity of fluorescence that moved toward the cell membrane was measured (see white lines in A and B), TMA and TMB showed comparable results, whereas TMD exhibited relatively distinct difference in the cytoplasm (denoted as C) and at the cell membrane (denoted as M). Also, fluorescence density at the cell membrane was higher than those of TMA and TMB. Therefore, all experiments were carried out using TMD which exhibited good translocation characteristic and translocation efficiency.

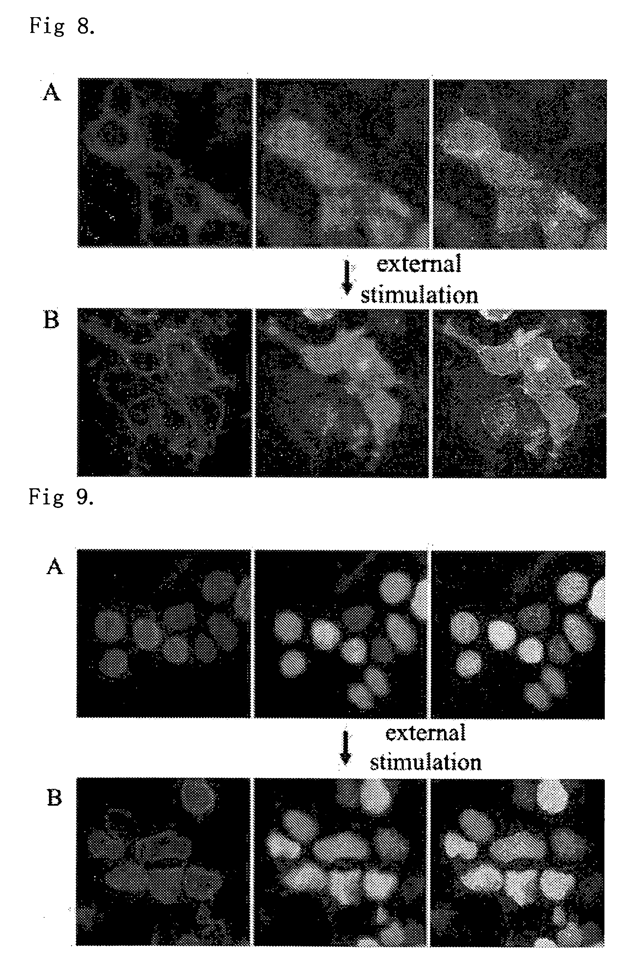

FIG. 8 shows the result of verifying the efficiency of NES sequence which limits the expression site of the first construct to the cytoplasm, in order to promote the efficacy of the present construct. In order to verify the effect of the NES sequence, a first construct (TMD-mRFP-Bait-NES) basic vector comprising the NES sequence (pTMD-mRFP-C3-NES vector) was prepared, and overexpressed in HEK-293 cell line along with a second construct (EGFP-Prey-NES) basic vector (pEGFP-C3-NES vector). The NES sequence-including first construct was observed mainly in the cytoplasm while the second construct was uniformly distributed in the whole cell (see A). After treating with PMA for 3 minutes, the first construct which had been uniformly distributed in the cytoplasm moved toward the cell membrane, whereas the second construct without including the NES sequence did not show any change in distribution (see B; left panel: first construct, central panel: second construct, right panel: merge).

FIG. 9 shows the result of verifying the efficiency of NLS sequence which limits the expression site of the first construct to the nucleus, in order to promote the efficacy of the present construct. In order to verify the effect of the NLS sequence, TMD-mRFP-Bait-NLS (pTMD-mRFP-C3-NLS vector) and EGFP-Prey-NLS (pEGFP-C3-NLS vector) basic vectors were prepared and overexpressed in HEK-293 cell line. The NLS sequence-including first construct and second construct were observed mainly in the nucleus (see A). After treating with PMA for 3 minutes, the first construct and second construct which had been uniformly distributed in the nucleus moved toward the nuclear membrane, whereas the second construct without the translocation module did not show any change in distribution (see B; left panel: first construct, central panel: second construct, right panel: merge).

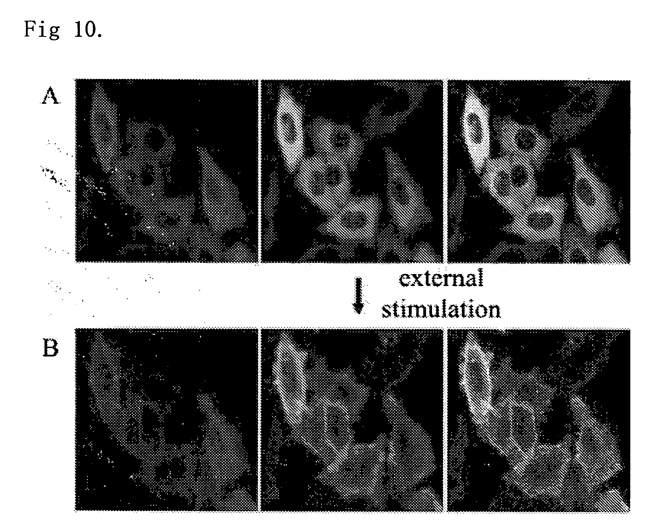

FIG. 10 shows the result of analyzing the binding of p53 protein and SV40T protein using the method of the present invention. In order to verify the binding of the bait (p53 protein) and the prey (SV40T protein), TMD-mRFP-p53 (first construct) and EGFP-SV40T (second construct) were prepared and overexpressed in CHO-k1 cell line. After treating with PMA for 3 minutes, the two proteins which had been uniformly distributed in the cell (A) moved toward the cell membrane (B) (red fluorescence: translocation module + p53 protein, green fluorescence: SV40T protein). Accordingly, it was confirmed that the two proteins are bound to each other in the cell (left panel: first construct, central panel: second construct, right panel: merge).

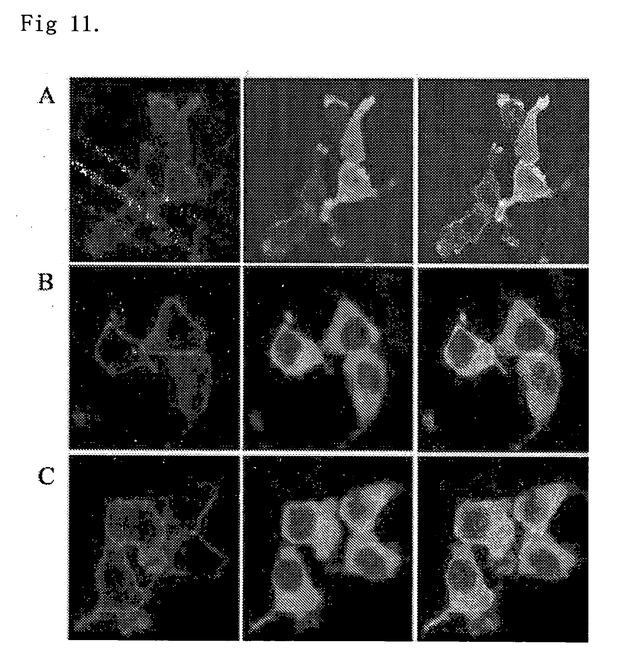

FIG. 11 shows the result of analyzing the binding of lysyl-tRNA synthetase (KRS) and JTV1 (p38), Gag and laminin receptor (LR). In order to verify the binding of p38, Gag and LR, which were expected to bind with the KRS protein,_KRS (as second construct) and p38, Gag and LR (as first construct) were overexpressed in HEK-293 cell line. Fluorescence distribution in the cell before and after treatment with PMA was analyzed. (A) TMD-mRFP-p38 and AzG-KRS strongly moved toward the cell membrane in response to an external stimulation, whereas TMD-mRFP-Gag (B) or TMD-mRFP-LR (C) labeled with green fluorescence did not show translocation. Accordingly, it was confirmed that KRS binds with p38 among the three proteins (left panel: first construct, central panel: second construct, right panel: merge).

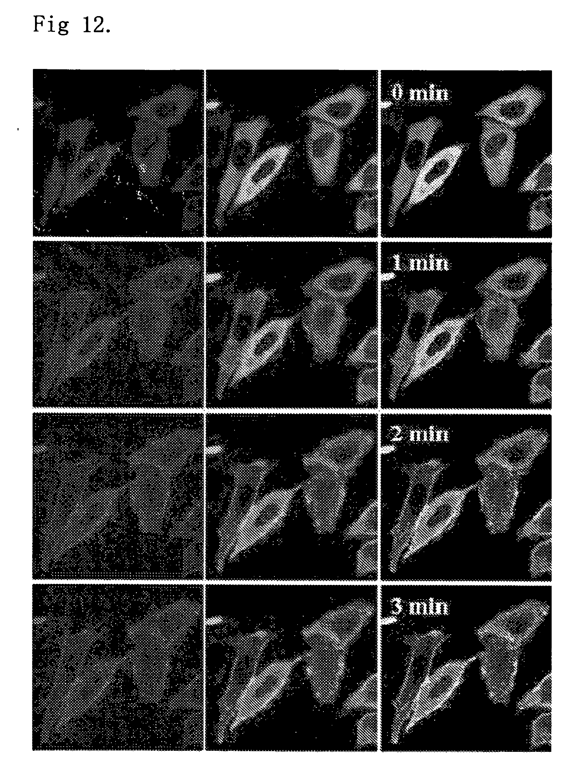

FIG. 12 shows the result of analyzing the binding of RelA and IkB in real time using a confocal laser fluorescence microscope. RelA and IkB, which are known to form a complex in a cell, were prepared into TMD-mRFP-RelA and EGFP-IkB, respectively, and overexpressed in CHO-k1 cell. Images were taken every 10 seconds, using a confocal laser fluorescence microscope. Prior to PMA treatment (0 minutes), fluorescences of the two proteins were uniformly distributed in the cytoplasm. About 10 seconds to 1 minute after the PMA treatment, the two fluorescences moved toward the cell membrane. Accordingly, it was confirmed that binding of two proteins in a living cell can be analyzed in real time. Hence, the present invention is useful for the study of cell signaling mechanisms in response to various stimulations including translocation signals (left panel: first construct, central panel: second construct, right panel: merge).

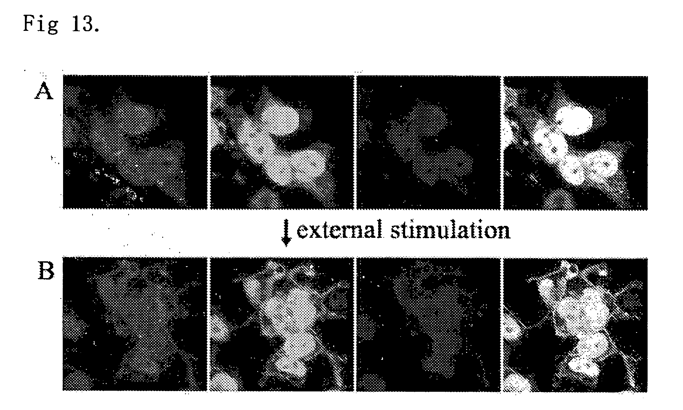

FIG. 13 shows the result of analyzing the binding of three protein complexes. The two proteins (RelA and IkB) of FIG. 12 are known to form an NFkB-IkB complex in a cell along with p50 protein. In order to verify whether the binding of a plurality of proteins can be analyzed, the second construct HcR-p50 was prepared by binding p50 protein with another fluorescent protein HcRed (HcR). The three constructs were overexpressed in HEK-293 cell line and their binding was observed using a confocal laser fluorescence microscope. Fluorescence signals (red, green and blue) reflecting the three proteins were uniformly distributed in the cytoplasm and nucleus prior to PMA treatment (A). However, after the PMA treatment (B), all of them moved toward the cell membrane. This result means that complexes composed of at least three proteins can be analyzed at the same time using the constructs of the present invention [from the left side, first panel: first construct (TMD-mRFP-RelA), second panel: second construct 1 (EGFP-IkB), third panel: second construct 2 (HcR-p50), right panel: merge].

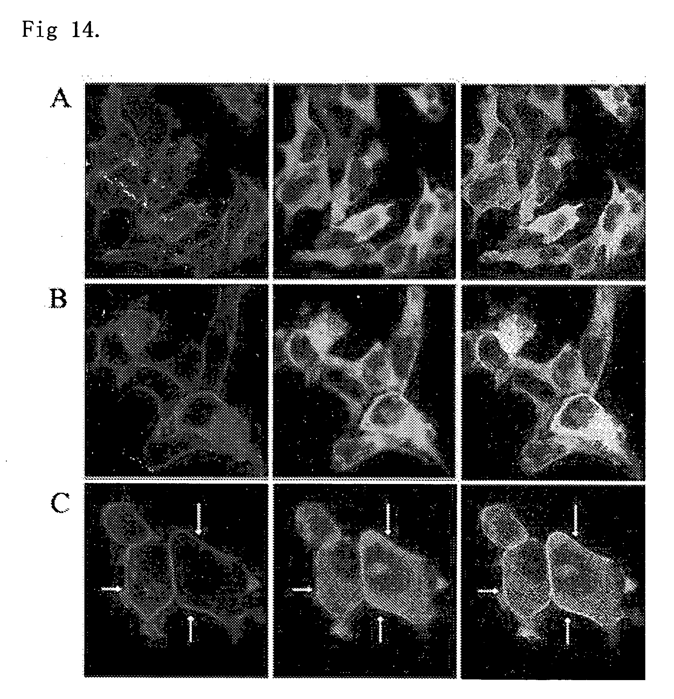

FIG. 14 shows the result of verifying the binding of positive candidates screened through Y2H. In order to reconfirm the binding of the candidates screened through Y2H, a screening method commonly used for screening of protein interaction, a first construct comprising OmpA protein as bait (TMD-mRFP-OmpA) and second constructs comprising EEF1A, FAM14B and DDX31 as prey candidates (EGFP-EEF1A, EGFP-FAM14B and EGFP-DDX31) were prepared. Combination of the bait and each of the prey candidates were overexpressed in HEK-293 cell line and their binding was observed using a confocal laser fluorescence microscope. Fluorescences were uniformly distributed in the cell prior to PMA treatment. After the PMA treatment, the translocation modules binding with the OmpA protein moved toward the cell membrane (red). Of the green fluorescences reflecting the prey candidates that were expected to bind thereto, EEF1A (A) and FAM14B (B) remained in the cytoplasm and DDX31 (C) moved toward the cell membrane. Thus, it was confirmed that only one of the three candidates screened through Y2H participates in the binding. This result shows that the present invention can solve the false positive problem of Y2H (left panel: first construct, central panel: second construct, right panel: merge).

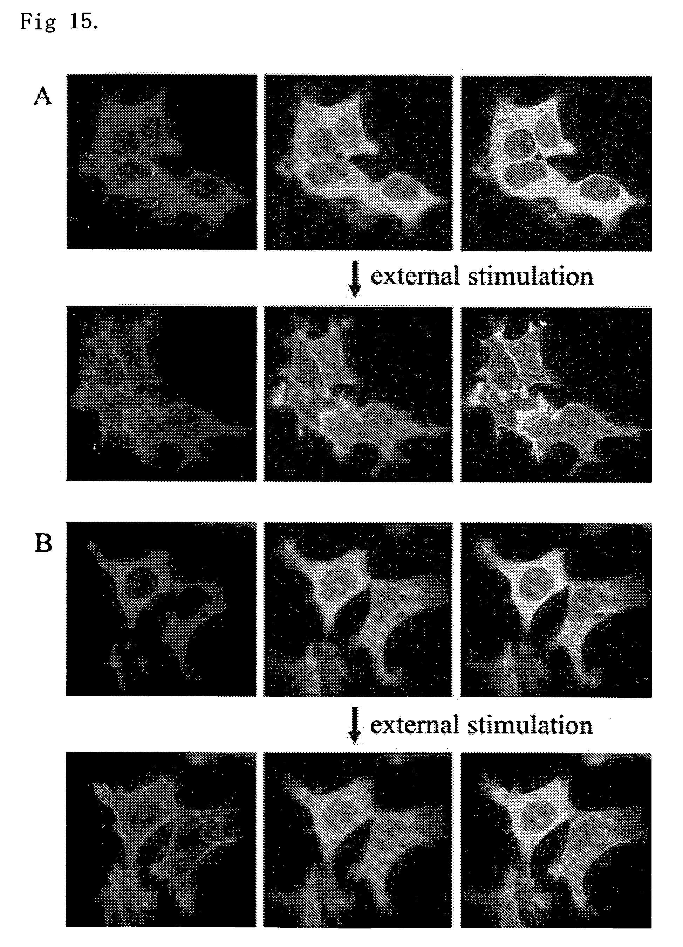

FIG. 15 shows the result of analyzing the binding of p53 protein with mdm2 protein and the inhibition of binding by the anticancer drug nutlin3. In order to verify the effectiveness of the present invention in new drug development, experiments were carried out on the inhibition of binding of an anticancer drug. To this end, a first construct comprising p53 protein (TMD-mRFP-p53N) and a second construct comprising mdm2 protein (AzG-mdm2N) were prepared. The constructs were overexpressed in HEK-293 cell line and their binding was observed using a confocal laser fluorescence microscope. First, in order to verify the binding of p53N and mdm2N, cells not treated with nutlin3 were compared before and after PMA treatment (A). The two proteins which had been uniformly distributed in the cytoplasm prior to the PMA treatment moved toward the cell membrane after the PMA treatment. This indicates that the two proteins are bound to each other. Next, in order to verify the binding inhibition effect of the anticancer drug nutlin3, cells were cultured in a medium containing 20 nM nutlin3 for 20 minutes. Then, fluorescence distribution was observed in real time before and after PMA treatment (B). In the nutlin3-pretreated cells, the first construct (red) comprising the translocation module and p53N moved toward the cell membrane, but the second construct (green) comprising only mdm2N and fluorescent protein with no translocation module did not show translocation toward the cell membrane. Thus, it was confirmed that the present invention is useful in verifying the inhibition of binding of p53 and mdm2 by the anticancer drug nutlin3. Therefore, the present invention can be used to screen inhibitors of binding of proteins or peptides (left panel: first construct, central panel: second construct, right panel: merge).

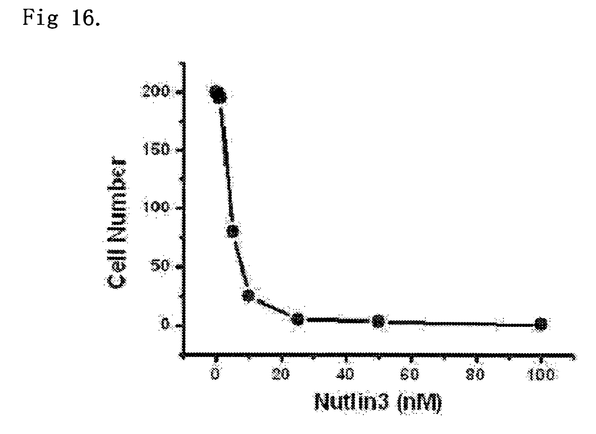

FIG. 16 shows the result of analyzing the binding inhibition concentration of the anticancer drug nutlin3. In order to determine the optimum concentration of the anticancer drug nutlin3, the binding inhibition effect of which was confirmed in FIG. 15, the binding inhibition effect was analyzed at various concentrations. Nutlin3 was treated at 0, 0.5, 1, 5, 10, 25, 50, 100, 200 nM. The binding inhibition effect of nutlin3 was distinct from 5 nM. At 10 nM, the binding between the two proteins was inhibited in more than 50% of the cells. At 25 nM, binding was not observed in more than 90% of the cells. This result shows that the binding inhibition effect of a binding inhibitor such as an anticancer drug can be precisely detected even at a very low concentration of 500 nM.

Accordingly, the present invention provides a method capable of detecting bindings and interactions occurring in a living cell in real time, and a method for screening a material that alters the interaction. The method of the present invention overcomes the disadvantages including inaccuracy and complexity of existing biomaterial interaction detection techniques, including in vitro method (in vitro and biochemical techniques), antibody binding techniques (antibody precipitation), fluorescence resonance energy transfer (FRET), bimolecular fluorescence complementation (Bi-FC) and fluorescence correlation spectroscopy (FCS) techniques, etc. By labeling both constructs to promote accuracy, the present invention provides a novel real-time, antibody-free analysis.

### EXAMPLES

Hereinafter, the present invention will be described in detail referring to the examples.

However, the following examples are for illustrative purposes only and are not intended to limit the scope of the present invention.

### <Example 1>

### Animal cell line and transformation

### <1-1> Animal cell line and culturing

CHO-k1 (ATCC CCL-61, *Cricetulus griseus*, hamster, Chinese), HEK293 (ATCC CRL-1573, *Hemo sapiens,* human), HeLa (ATCC CCL-2, *Homo sapiens,* human) and SH-SY5Y (ATCC CRL-2266, *Homo sapiens,* human) cell lines were used. The animal cells were cultured according to the instructions of ATCC (American Type Culture Collection) for the individual cells. CHO-k1 cells were cultured using F-12 medium, and HEK293, HeLa and SH-SY5Y cells were cultured using DMEM medium. Other culturing condition was the same. The cells were cultured as follows (Those skilled in the art may modify the specific conditions depending on purposes.). The cells were cultured in pH 7.4 medium (F-12 and DMEM) containing 25 mM HEPES, 10% fetal bovine serum (FBS, v/v), 100 units/ml penicillin and 100 µg/ml streptomycin in a 5% CO₂ incubator maintained at 37 °C.

### <1-2> Transformation of cell line

In the Examples of the present invention, genes were introduced into the cells using ExGene 500 (Fermentas Life Science), one of the liposome-based techniques. All the conditions for the gene introduction including gene concentration were pursuant to the manufacturer's instructions. More specifically, after transferring the subcultured cells to a 12-well plate with a cover slip, followed by culturing for a day, the culture medium was replaced with 0.9 ml of fresh medium. About 1 µg of the transformation sample was added to 0.1 ml of 150 mM NaCl solution. After completely mixing, 3.3 µl of ExGene reagent was added and mixed by vortexing for 15 seconds. The resultant solution was allowed to stand at room temperature for 10 minutes and then added to each well of the 12-well plate in which the cells were growing. The cells were allowed to be transformed by culturing for 18 hours.

### <Example 2>

### Design and preparation of first construct and second construct

### <2-1> Design and preparation of first construct

The first construct may be fused construct comprising a translocation module capable of moving the protein uniformly expressed in the cytoplasm toward the cell membrane, a fluorescent protein analyzable using a microscope, and a bait.

Vectors expressing the first construct (see A of FIG. 4) were prepared as follows. Translocation modules were prepared by PCR using the following templates and primers and inserted at the NheI/AgeI site of a pEGFP-C3 vector (GenBank Accession No. U57607; Clontech Catalog No. #6082-1, SEQ ID NO: 21) and a pmRFP-C3 vector (mRFP; GenBank Accession No. DQ903889, SEQ ID NO: 22), thereby completing the vectors.

The TMD translocation module was prepared by PCR using a pCMV-SPORT6-PRKCD vector [GenBank Accession No. BC043350; purchased from Open Biosystems (http://www.openbiosystems.com/); Catalog No. EHS1001-410108-BC043350] as template and SEQ ID NO: 23 (PRKCD-F; 5'-GAAGCTAGCCGCCACCATGGCGCCGTTCCTGC-3') and SEQ ID NO: 24 (PRKCD-R; 5'-GAAACCGGTGGATCTTCCAGGAGGTGCTCGAATTTGG-3') as primers. And, the TMA translocation module was prepared by PCR using a pCMV-SPORT6-PRKCD vector as template and SEQ ID NO: 25 (TMA-F; 5'-GAAGCTAGCCGCCACCATGAAACAGGCCAAAATCCACTACATC-3') and SEQ ID NO: 26 (TMA-R; 5'-GAAACCGGTGGAGTGTCCCGGCTGTTGGCCGC-3') as primers. Further, the TMB translocation module was prepared by PCR using a pCMV-SPORT6-PRKCD vector as template and SEQ ID NO: 27 (TMB-F; S'-GCAGCTAGCCGCCACCATGCAGAAAGAACGCTTCAACATCG-3') and SEQ ID NO: 28 (TMB-R; 5'-GCAACCGGTGGGGCCTCAGCCAAAAGCTTCTG-3') as primers.

### <2-2> Design and preparation of mutated first construct

Since the translocation module included in the first construct is derived from a protein kinase, it intrinsically has the function of phosphorylation. There is a risk that cell signaling and protein interaction in the cells where the constructs are overexpressed may be inhibited or interfered by the intrinsic function of phosphorylation. Accordingly, in the present invention, mutation is induced to deprive the phosphorylation of the translocation module. In order to substitute the 311 st amino acid (tyrosine) and the 378th amino acid (lysine) of the kinase used in the present invention, which are known as very important phosphorylation sites, with phenylalanine (Y313F) and arginine, respectively, mutagenesis by PCR was performed and, finally, TMD translocation module was prepared.

To this end, genes were amplified using a forward primer (PKCD-F; 5'-GAAGCTAGCCGCCACCATGGCGCCGTTCCTGC-3', SEQ ID NO: 29) and a modified reverse primer (Y313F-R; 5'-GAAACCCTGA**A**ATATCCCAAC-3', SEQ ID NO: 30) under a first PCR condition, and then genes were amplified using a modified forward primer (Y313F-F; 5'-GTTGGGATAT**T**TCAGGGTTTC-3', SEQ ID NO: 31) and a reverse primer (PKCD-R; 5'-GAAACCGGTGGATCTTCCAGGAGGTGCTCGAATTTGG-3', SEQ ID NO: 32) under a second PCR condition. The PCR products obtained from the first and second PCR were used as template for a third PCR using forward and reverse primers. The resultant gene is a Y313F mutant wherein the nucleotide sequence TAT is substituted by TTT. Using the gene as template, a K378R mutant wherein AAG is substituted by AGG was prepared through the same experimental procedure, using a forward primer (SEQ ID NO: 29), a reverse primer (SEQ ID NO: 32), a modified forward primer (K378R-F: 5'-TTTGCCATCA**G**GGCCCTCAAG-3', SEQ ID NO: 33) and a modified reverse primer (K378R-R: 5'-CTTGAGGGCC**C**TGATGGCAAA-3', SEQ ID NO: 34).

### <2-3> Adhesion of NLS and NES to first construct

Since the proteins existing in the organisms have their own distribution characteristics (targeting), the bait and prey may have targeting sites in the cell organelles. In this case, the detection of the change of the location of the first construct and second construct may be difficult. Accordingly, there is a need to control the distribution to the cytoplasm or nucleus so that the binding between the bait and prey can be verified through experiments. To this end, a vector capable of controlling the locational distribution of the bait and prey was prepared using a nuclear exclusion signal (NES) which targets a protein to the cytoplasm and a nuclear localization signal (NLS) which targets a protein to the nucleus.

The vector for locational control makes it possible to recognize whether the binding between the bait and prey occurs in the cytoplasm or in the nucleus. That is, assuming that the bait and prey bind in the cytoplasm and move toward cytoplasm, a combination of a first construct and a second construct both containing an NES allows the second construct to bind with the first construct and to move toward the cell membrane by PMA treatment, whereas a combination of constructs both containing an NLS allows only the first construct to move toward the nuclear membrane, not showing the movement of the second construct. In contrast, assuming that the bait and prey bind only in the nucleus, a combination of constructs both containing an NLS allows the constructs to move toward the nuclear membrane by PMA treatment, whereas a combination of constructs both containing an NES allows only the first construct to move toward the cell membrane, not showing the movement of the second construct.

The first construct and second construct comprising an NLS were prepared through a series of conventional PCR cloning method using the following primers. A first PCR was carried out using a pmRFP-C3 vector as template and using SEQ ID NO: 35 (NLS-F-1: 5'-AGTAAAAAGGAAAAGGATAAATAGATAACTGATCATAATCAGCC-3') and SEQ ID NO: 36 (NLS-R : 5'-GCTGCAATAAACAAGTTAACAAC-3') as primers. A second PCR was carried out using the resultant PCR product as template and using SEQ ID NO : 37 (NLS-F-2: 5'-TGGAAGAAGTAGCTAAGAAGAAGAGTAAAAAGGAAAAGGATAAA-3') and SEQ ID NO: 36 (NLS-R) as primers. Similarly, a third PCR was carried out using SEQ ID NO : 38 (NLS-F-3: 5'-TCCGGTGATGAAGTCGAAGGAGTGGAAGAAGTAGCTAAGAAGAA-3') and SEQ ID NO: 36 (NLS-R) as primers, and a fourth PCR was carried out using SEQ ID NO: 39 (NLS-F-4: 5'-GCTGGATCCAGGCTCTGGTGATGAAGTCGAAGG-3') and SEQ ID NO: 36 (NLS-R). Thus obtained gene fragments were inserted at the BamHI/HpaI site of the first construct vector or the second construct vector.

The first construct and second construct comprising an NES were prepared in the same manner, using the following primers. A first PCR was carried out using a pmRFP-C3 vector as template and using SEQ ID NO: 40 (NES-F-1: 5'-GTGGGAAACATTTCCCTGGTGTAGATAACTGATCATAATCAGCC-3') and SEQ ID NO: 41 (NES-R: 5'-GCTGCAATAAACAAGTTAACAAC-3') as primers. A second PCR was carried out using the resultant PCR product as template and using SEQ ID NO: 42 (NES-F-2: 5'-GTCATCATCAAGCTGAACGCCCATGTGGGAAACATTTCCCTGGT-3') and SEQ ID NO: 41 (NES-R) as primers. Similarly, a third PCR was carried out using SEQ ID NO : 43 (NES-F-3: 5'-GTCGGATCCAGACCAGCGCGTCATCATCAAGCTGAACGCC-3') and SEQ ID NO: 41 (NES-R) as primers. Thus obtained gene fragments were inserted at the BamHI/HpaI site of the first construct vector or the second construct vector.

### <2-4> Design and preparation of second construct

The second construct comprises a labeling material for analyzing the movement of the prey which has characteristic for binding with the bait of the first construct. The second construct was prepared using a fluorescent material other than used in the first construct. Using green fluorescent protein (EGFP, AzG), red fluorescent protein (mRFP) and infrared fluorescent protein (HcR), the second construct was prepared by the method described for the first construct.

When EGFP was used as the second labeling material, a pEGFP-C3 vector (Clontech) was used. When mRFP was used, a pmRFP-C3 vector was used. When AzG was used, a pAzG-C3 vector was used. And, when HcR was used, a pHcR-C3 vector was used. The C3 vectors had been prepared by substituting the EGFP gene sequence site of the pEGFP-C3 vector with AzG and HcR genes, as follows.

The pAzG-C3 vector was prepared as follows. PCR was carried out using a pPM-mAGI vector (purchased from MBL, Catalog No. AM-V0203; Karasawa, S., et al. 2003, J. Biol. Chem. 278, 34167-34171) as template and using SEQ ID NO: 44 (AzG-F: 5'-GGCACCGGTCGCCACCATGGACCCCATGGTGAGTGTGAT-3') and SEQ ID NO: 45 (AzG-R: 5'-GGCAGATCTGACAGCTTGGCCTGACTCGGCAGCAT-3') as primers. Then, the EGFP nucleotide sequence of the pEGFP-C3 vector was substituted at the AgeI/NotI site by the resultant PCR product.

The pHcR-C3 vector was prepared as follows. PCR was carried out using pHcRed-Tandem-NI (purchased from Avrogen, Catalog No. FP204; Gurskaya et al., 2001, FEBS Lett. 507, 16-20.) as template and using SEQ ID NO: 46 (HcR-F: 5'-GCCACCGGTCGCCACCATGGTGAG-3') and SEQ ID NO: 47 (HcR-R: 5'-GCCGCGGCCGCTTATCAGTTGGCCTTCTCGGGCAGGTC-3') as primers. Then, the EGFP nucleotide sequence of the pEGFP-C3 vector was substituted at the AgeI/NotI site by the resultant PCR product.

### <Example 3>

### Verification of translocation characteristics of first construct and second construct

### <3-1> Verification of expression of constructs and analysis of translocation characteristics

A cover slip containing the cells in which the first construct and second construct vectors had been introduced was fixed to a perfusion chamber and mounted on the object stage of a confocal laser fluorescence microscope (Carl Zeiss LSM510). Images of the construct vectors were taken before and after external stimulation (treatment with 1 µM PMA).

488 nm argon laser (EGFP or AzG), 543 nm HeNe laser (mRFP) or 561 nm DPSS laser (HcR) of the confocal laser fluorescence microscope was used to excite the fluorescent label, and the fluorescence signal generated by each fluorescent label was filtered through the band path filter BP505-530 (EGFP or AzG), long path filter LP560 or BP560-630 (mRFP) or long path filter LP650 (HcR). Images were taken after completely removing the interference between the fluorescences.

As a result, the green or red fluorescence emitted by the first construct vector comprising the translocation module (TMD) moved toward the cell membrane (see FIG. 5 A), whereas the green or red fluorescence emitted by the second construct vector with no translocation module was uniformly distributed in the cytoplasm as before the stimulation (see FIG. 5 B). Accordingly, it can be seen that the second construct vector docs not respond to the external stimulation and that the movement of the second construct toward the cell membrane necessarily requires the binding of the bait and prey.

### <3-2> Verification of translocation of first construct and optimum concentration of external stimulant

Translocation efficiency of the first construct using the pTMD-mRFP-C3 vector depending on the concentration of external stimulant PMA was measured as follows. The expression vector of the first construct was transformed into CHO-kl cells and overexpressed. After 18 hours of culturing, PMA was treated at 1, 5, 10, 20, 40, 50, 80 and 100 nM and 1 and 5 µM. 5 minutes after the PMA treatment, the cells were fixed with 3.8% formaldehyde and observed using a confocal microscope (Carl Zeiss LSM 510). 200 cells exhibiting red fluorescence were randomly selected and the distribution of the red fluorescence at the cell membrane was measured for each concentration.

As a result, as seen in FIG. 6, translocation was observed in 90% or more of the cells at 50 nM. At 100 nM or above, translocation of the red fluorescence toward the cell membrane increased. Therefore, it can be seen that treatment at a concentration of 50 nM to 5 µM is preferred, but treatment at 1 µM, which is a concentration sufficient for the response and movement of most cells, is effective.

### <Example 4>

### Analysis of binding in cell in real time using first construct and second construct

### <4-1> Analysis of binding between p53 protein and SV40T antigen in cell

For the binding between p53, which is known as a potential carcinogenic gene, and SV40T antigen, which is known to bind to p53, there is a commercialized binding screening system based on IP. Analysis was carried out using the same proteins in order to verify the basic binding detection ability of the present invention. In order to verify the binding between p53 and SV40T antigen in cell, a first construct in which the p53 protein is fused (TMD-mRFP-p53) and a second construct in which the SV40T antigen is fused were prepared as follows.

The first construct TMD-mRFP-p53 was prepared as follows. PCR was carried out using a pGBK-p53-GAL4 vector (p53; GenBank Accession No. AF161020) as template and using SEQ ID NO: 48 (p53-F: 5'-GAAGAATTCTGATGCCTGTCACCGAGACCCCTGGG-3') and SEQ ID NO: 49 (p53-R: 5'-GAAGGATCCCGTCAGTCTGAGTCAGGCCCCACTT-3') as primers. The resultant PCR product was inserted at the EcoRI/BamHI site of the pTMD-mRFP-C3 vector (a vector obtained by inserting a TMD sequence into an mRFP-C vector, see Example 2).

The second construct EGFP-SV40T was prepared as follows. PCR was carried out using a pGADT7-SV40T-GAL4 vector (SV40T; GenBank Accession No. BC014270) as template and using SEQ ID NO: 50 (SV40T-F: 5'-GAAGAATTCTGATGGGAACTGATGAATGGGAGCAG-3') and SEQ ID NO: 51 (SV40T-R: 5'-GAAGGATCCCGTTATGTTTCAGGTTCAGGGGG-3') as primers. The resultant PCR product was inserted at the EcoRI/BamHI site of the pEGFP-C3 vector.

The first construct (TMD-mRFP-p53) and the second construct (EGFP-SV40T) were introduced into CHO-k1 cells according to the procedure described in Example <1-2> (ExGene 500) and allowed to be expressed. After 18 hours of culturing, followed by treatment with 1 µM PMA for 5 minutes, fluorescence distribution of the two constructs was observed as in Example <3-1>.

As seen in FIG. 10, both the red fluorescence (left panel) emitted by the bait p53 protein bound to the translocation module and the green fluorescence (central panel) emitted by the prey SV40T moved toward the cell membrane after the PMA treatment. In contrast, considering that translocation toward the cell membrane did not occur in a control group wherein only the second construct not bound to SV40T was expressed (result not shown), it can be seen that the prey SV40T binds with the bait p53 in the cell. Also, because the change of distribution of prey was appeared due to the change of distribution of bait, to which a translocation module was attached, it is confirmed that the two proteins bind to each other in cell.

### <4-2> Analysis of binding between KRS protein and p38(AIMP2) protein

Lysyl-tRNA synthetase (KRS), known as a multifunctional pathogenic gene, is known to form a complex with p38/AIMP2 protein and at least 3 other aminoacyl-tRNA synthetases (ARSs) (J. Cell Science, 2004, 117, 3725-3734, references therein). Accordingly, in order to verify the possibility of analyzing the binding of KRS protein and p38 protein in a cell in real time, the applicability in various animal cells, the diversity of fluorescent labels, and the exchangeability of the bait and prey, analysis was carried out using p38, which is known to bind with KRS, and using Gag and LR, which are expected as potential binding proteins.

To this end, a first construct (TMD-mRFP-p38, TMD-mRFP-Gag or TMD-mRFP-LR) and a second construct (AzG-KRS) were prepared as follows. AzG was used instead of EGFP as the fluorescence for the second construct.

The first construct TMD-mRFP-p38 was prepared as follows. PCR was carried out using a pGEX-4T1-p38 vector (p38; GenBank Accession No. NM_006303) as template and using SEQ ID NO: 52 (p38-F: 5'-GTCCTCGAGATGCCGATGTACCAGGTAAAG-3') and SEQ ID NO: 53 (p38-R: 5'-GTCGGATCCTTAAAAAGGAGCCAGGTTTTC-3') as primers. The resultant PCR product was inserted at the XhoI/BamHI site of the pTMD-mRFP-C3 vector.

TMD-mRFP-Gag was prepared as follows. PCR was carried out using a pGEX-4T1-Gag vector (Gag; GenBank Accession No. NM_002295) as template and using SEQ ID NO: 54 (Gag-F: 5'-GTCGAATTCTGATGGGTGCGAGAGCGTCAGTA-3') and SEQ ID NO: 55 (Gag-R: 5'-GTCGGATCCTTATTGTGACGAGGGGTCGTT-3') as primers. The resultant PCR product was inserted at the XhoI/BamHI site of the pTMD-mRFP-C3 vector.

TMD-mRFP-LR was prepared as follows. PCR was carried out using a pET28a-TEV-LR vector (LR; GenBank Accession No. NM_002295) as template and using SEQ ID NO: 56 (LR-F: 5'-GTCGAATTCTGATGTCCGGAGCCCTTGATGT-3') and SEQ ID NO : 57 (LR-R: 5'-GTCGGATCCTTAAGACCAGTCAGTGGTTGCTC-3') as primers. The resultant PCR product was inserted at the XhoI/BamHI site of the pTMD-mRFP-C3 vector.

The second construct AzG-KRS was prepared as follows. PCR was carried out using pET28a (GenBank Accession No. NM_005548) as template and using SEQ ID NO: 58 (KRS-F: 5'-GTCGAATTCTGATGGCGGCCGTGCAGGCG-3') and SEQ ID NO: 59 (KRS-R: 5'-GTCCCCGGGCTAGACAGAAGTGCCAACTGTTGTG-3') as primers. The resultant PCR product was inserted at the EcoRI/BamHII site of the pAzG-C3 vector.

Thus prepared first construct and second construct were transformed into HEK293 cell line. Fluorescence was observed as in the same manner as in Example <4-1> except for using DMEM.

As seen in FIG. 11, all the three proteins of the first construct were observed to move toward the cell membrane (left panel). As for p38 (see FIG. 11 A), which is known to bind to KRS, the second construct bound to KRS moved toward the cell membrane. However, such translocation was not observed for the potential binding proteins Gag (see FIG. 11 B) and LR (see FIG. 11 C) (central panel). Accordingly, it was verified that KRS protein binds with p38 protein but not with Gag or LR protein. Further, it was verified that AzG fluorescent protein may be used instead of EGFP.

### <4-3> Analysis of binding between RelA protein and inhibitor protein IkB in cell in real time

It is known that the interactions of NFkB and IkB proteins regulated by TNF-alpha are involved with various cell signaling pathways occurring in cells. Of the various cell signaling pathways, particularly noticeable is the close relationship with inflammatory responses against various harmful signals in body. Accordingly, in order to verify the binding of the multifunctional inflammation-related gene RelA and the inhibitor protein IkB, among the NFkB complexes, and to demonstrate the possibility of direct analysis in living cells after external stimulation, not by cell fixation, experiments were carried out using a real-time (time-laps) technique.

To this end, a first construct (TMD-mRFP-RelA) and a second construct (EGFP-IkB) were prepared as follows.

The first construct TMD-mRFP-RelA was prepared as follows. PCR was carried out using a pEYFP-RelA vector (RelA; GenBank Accession No. NM_021975) as template and using SEQ ID NO: 60 (RelA-F: 5'-GGACTCGAGATGGACGAACTGTTCCCCCTC-3') and SEQ ID NO: 61 (RelA-R: 5'-GAAGGATCCCGTTAGGAGCTGATCTGACTCAGCAGG-3') as primers. The resultant PCR product was inserted at the XhoI/BamHI site of the pTMD-mRFP-C3 vector.

The second construct EGFP-IkB was prepared as follows. PCR was carried out using a pcDNA3-IkB vector (IkB; GenBank Accession No. NM_020529) as template and using SEQ ID NO: 62 (IkB-F: 5'-GAAGAATTCTGATGTTCCAGGCGGCCGAGCG-3') and SEQ ID NO: 63 (IkB-R: 5'-GAAGGATCCCGTCATAAACGTCAGACGCTGGCCTCCAA-3') as primers. The resultant PCR product was inserted at the EcoRI/BamHI site of the pEGFP-C3 vector.

Thus prepared first construct and second construct were transformed into CHO-k1 cell line and fluorescence was observed as in Example <4-1> at 0, 1, 2 and 3 minute.

As seen in FIG. 12, the first construct (TMD-mRFP-RelA) was uniformly distributed in the cell at 0 minute, but moved gradually toward the cell membrane with the lapse of time (left panel). The second construct (EGFP-IkB) was also uniformly distributed in the cell at 0 minute, but moved gradually toward the cell membrane along with the first construct with the lapse of time (central panel). Especially, the constructs were observed to move toward the cell membrane from at about 10 seconds after the PMA treatment (not shown in the figure). The translocation was completed in most cells within 3 minutes. This tendency was observed similarly in all the preceding examples and all the following examples (not shown in the figure).

### <4-4> Analysis of binding of multifunctional inflammation-related gene NFkB complex (ReIA/p50/IkB) in cell

Basically, the NFkB complex is known to be formed by NFkB (ReIA/p50) and the negative regulator IkB protein. It was verified whether the binding of the three proteins RelA, p50 and IkB, which constitute the complex, can be identified.

To this end, a first construct (TMD-mRFP-RelA) and two second constructs (EGFP-IkB and HcR-p50) were prepared as follows. As the fluorescent label, mRFP was used for the first construct, and EGFP and HcR (HcRed), which is distinguishable to mRFP, were used for the second constructs.

The first construct (TMD-mRFP-RelA) and the IkB-comprising second construct (EGFP-IkB) were the same as those used in Example <4-3>. The other second construct HcR-p50 was prepared as follows. PCR was carried out using a pDMV-SPORT6-NFkB1 vector (NFkB1 GenBank Accession No. BC006231) as template and using SEQ ID NO: 64 (p50-F: 5'-GCTGAATTCTGATGGCAGAAGATGATCCATATT-3') and SEQ ID NO: 65 (p50-R: 5'-GCTCCCGGGCTTAATGCTTCATCCCAGCATTAGA-3') as primers. The resultant PCR product was inserted at the EcoRI/XmaI site of the pHcR-C3 vector.

Thus prepared first construct and two second constructs were transformed into HEK293 cell line, and fluorescence was observed as in Example <4-1>.

As seen in FIG. 13, all the first construct (TMD-mRFP-RelA, red), the second construct (EGFP-IkB, green) and the second construct (HcR-p50, blue) moved toward the cell membrane in response to the PMA stimulation. In contrast, the change of distribution was not observed in the control group (TMD-mRFP) wherein the first construct did not include a translocation module (not shown in the figure).

This experimental result indicates that not only the binding of the three protein complexes comprising the first construct, but also the binding of four complexes EGFP, mRFP, HcRed and BFP with the fluorescent label removed from the first construct can be verified using a generally used fluorescence microscope or confocal laser fluorescence microscope. Using various fluorescent protein species and the Meta (Carl Zeiss) or Spetral (Leica) fluorescence microscope, at least five protein complexes including BFP, CFP, GFP, RFP and Far-Red can be detected in addition to the first construct.

### <Example 5>

### Verification of candidate materials screened through Y2H

Currently, Y2H is the most widely used for screening protein bindings in a living cell. It is also the typical method used for new drug screening. However, Y2H is disadvantageous in new drug targeting and verification because of the high false positive and the use of yeast. Accordingly, the applicability of the present invention as a method of verifying the candidate materials screened through Y2H was investigated.

Four positive clones were screened through Y2H for super bacteria (antibiotics-resistant bacteria)-related OmpA (GenBank Accession No. AY485227) protein and human protein library (Entire library screening clones: 1.188×10⁵ clones; first patch/streak screening: 137 clones; second re-transformation screening: 54 clones; third prey auto-activation test screening: 14 clones; fourth nucleotide sequencing confirmation: 4 clones).

Among the four positive clones, EEF1A1 (GenBank Accession No. BC009875), FAM14B (GenBank Accession No. BC015423) and DDX31 (GenBank Accession No. AK027002) were tested for binding with the OmpA protein. To this end, a first construct (TMD-mRFP-OmpA) and second constructs (EGFP-EEF1A, EGFP-FAM14B and EGFP-DDX31) were prepared as follows.

The first construct TMD-mRFP-OmpA was prepared as follows. PCR was carried out using a pET28a-OmpA (GenBank Accession No.: AY185227) vector as template and using SEQ ID NO: 66 (OmpA-F: 5'-GCTGAATTCTGATGAAATTGAGTCGTATTGCAC-3') and SEQ ID NO: 67 (OmpA-R: 5'-GCTGGATCCTTATTGAGCTGCTGCAGGAGC-3') as primers. The resultant PCR product was inserted at the EcoRI/BamHI site of the pTMD-mRFP-C3 vector.

The second constructs were prepared as follows. For EGFP-EEF1A, a pOTB7-EEF1A (GenBank Accession No. BC009875) vector was used as template and SEQ ID NO: 68 (EEF1A-F: 5'-GCTGAATTCTGATGGGAAAGGAAAAGACTCA-3') and S E Q I D N O : 69 (EEF1A-R: 5'-GCTGGATCCCGCTATTTAGCCTTCTGAGCTT-3') were used as primers. For EGFP-FAM14B, a pCMV-SPORT6-FAM14B(GenBank Accession No.: BC015423) vector was used as template and SEQ ID NO: 70 (FAM14B-F: 5'-GTCGAATTCTGATGGGAAAGGAGAGTGGATGG-3') and SEQ ID NO: 71 (FAM14B-R: 5'-GTCGGATCCCGTCAGCTGGAAGGGGGTGAAC-3') were used as primers. For EGFP-DDX31, a pME18S-FL3-DDX31 vector was used as template and SEQ ID NO: 72 (DDX31-F: 5'-GTCGAATTCTGATGTTTTCTCCAAAGAAGCAT-3') and SEQ ID NO: 73 (DDX31-R: 5'-GTCGGATCCCGTTAAACTTTCTGGGAAGTCTTG-3') were used as primers. After carrying out PCR, each PCR product was inserted at the EcoRI/BamHI site of the pEGFP-C3 vector.

Thus prepared first construct and second constructs were transformed into HEK293 cell line, and fluorescence was observed as in Example <4-1>.

As seen in FIG. 14, EEF1A1 (A) and FAM14B (B) did not move toward the cell membrane along with the OmpA-bound first construct (false positive). In contrast, DDX31 (C) moved toward the cell membrane along with the OmpA-bound first construct, and thus, was confirmed as positive. Therefore, it can be seen that the method of the present invention provides better accuracy than Y2H and enables reconfirmation of positive binding.

### <Example 6>

### Verification of efficiency of anticancer drug through real-time analysis

p53 protein, known as a potential carcinogenic protein, is known to facilitate carcinogenesis by binding at least to mdm2 protein. Through researches, nutlin3 was confirmed as a potent anticancer drug capable of inhibiting it. In an in vitro binding inhibition experiment using Biacore's surface plasmon resonance (SPR) technology, a p53-mdm2 binding inhibition effect of 90% or more was attained when at least 1 micromolar of nutlin3 was treated, and the median inhibitory concentration (IC50) was about 90 nanomolar (Vassilev, L. T. et. al., 2004, Science 303, 844-848).

The interaction between p53 (GenBank Accession No. NM_00546) and mdm2 and the binding inhibition by nutlin3 were verified in living human cell line. To this end, a first construct (TMD-mRFP-p53N) and a second construct (AzG-mdm2N) were prepared as follows.

The first construct TMD-mRFP-p53N was prepared as follows. PCR was carried out using a pGEX-4T1-p53N vector (p53; GenBank Accession No. NM_000546) as template and using SEQ ID NO : 74 (p53N-F: 5'-GTCGAATTCTCATGGAGGAGCCGCAGTCAGAT-3') and SEQ ID NO: 75 (p53N-R: 5'-GTCGGATCCTCACACGGGGGGAGCAGCCT-3') as primers. The resultant PCR product was inserted at the EcoRI/BamHI site of the first construct vector (pTMD-mRFP-C3 vector).

The second construct EGFP-mdm2N was prepared as follows. PCR was carried out using a pGEX-4T1-mdm2N vector (mdm2; GenBank Accession No. NM_002392) as template and using SEQ ID NO: 76 (mdm2N-F: 5'-GTCGAATTCTGATGTGCAATACCAACATGTCTGTACC-3') and SEQ ID NO: 77 (mdm2N-R: 5'-GTCGGATCCTCATACTACCAAGTTCCTGTAGAT-3') as primers. The resultant PCR product was inserted at the EcoRI/BamHI site of the pAzG-C3 vector.

Thus prepared first construct and second construct were transformed into HEK393 cell line, and fluorescence was observed as in Example <4-1>. Nutlin3 was treated at 0, 0.5, 1, 5, 10, 25, 50, 100 and 200 nM. As in Example <3-2>, after treating with nutlin3 for 5 minutes, the cells were fixed with 3.8% formaldehyde and observed using a confocal microscope (Carl Zeiss LSM 510). 200 cells exhibiting red fluorescence were randomly selected and the distribution of the red fluorescence at the cell membrane was measured for each concentration.

As seen in FIG. 15, mdm2 (green, central panel) moved toward the cell membrane in a living cell along with p53 (red, left panel) in response to the external stimulation (PMA) (see FIG. 15 A), whereas a change in distribution was not observed when the inhibitor nutilin3 was treated (see FIG. 15 B). This indicates that mdm2 binds with p53 in the absence of nutilin3, but the binding of mdm2 and p53 is inhibited by nutilin3. This result shows that the present invention enables a direct analysis of protein interactions occurring in a living cell and may be useful as a tool for new drug development associated with the bindings.

Also, as seen in FIG. 16, by the result that 200 cells exhibiting red fluorescence were randomly selected and the distribution of the red fluorescence at the cell membrane was measured for each concentration, a binding inhibition effect of 95% or more was attained at 20 nanomolar (nM), whereas a binding inhibition effect of 90% or more was attained at 1 micromolar in an in vitro experiment for living human cells (HEK293) using recombinant proteins (Vassilev, L. T. et. al., 2004, Science 303, 844-848). Accordingly, it can be seen that the present invention provides an analysis accuracy of about 50 times that of the existing in vitro technique. In addition, this result indicates that the present invention provides a technique capable of avoiding the risk of research for cells or body and clinical tests for humans, by the in vitro inhibitory result.

### INDUSTRIAL APPLICATION

As described, the present invention provides a method enabling real-time detection of bindings and interactions of materials occurring in a living cell and a method for screening a material altering such interactions. The method of the present invention overcomes the disadvantages including inaccuracy and complexity of existing biomaterial interaction detection techniques, including in vitro method (in vitro and biochemical techniques), antibody binding (antibody precipitation) techniques, FRET, Bi-FC and FCS techniques, etc. By labeling both constructs to promote accuracy, the present invention provides a novel real-time, antibody-free analysis.

## Claims

1. A method for detecting interactions of a polypeptide bait and a polypeptide prey comprising the steps of:
(a) preparing a cell comprising (i) a first fused construct comprising a polypeptide bait, a first labeling material and a translocation module having an amino acid sequence represented by SEQ ID NO:7; and (ii) a second fused construct comprising a polypeptide prey and a second labeling material;
(b) inducing a translocation of the translocation module via an internal signaling mechanism or by treating with a signaling material that generates an external signal; and
(c) detecting a distribution of the first construct and the second construct in the cell, wherein the first labeling material is a fluorescent protein, wherein the second labeling material is a fluorescent protein, wherein the first and the second labeling materials are different for distinction.

2. The method of claim 1 for detecting interactions of a polypeptide bait and a polypeptide prey comprising the steps of:
(a) preparing a cell comprising (i) a first fused construct comprising a polypeptide bait, a first labeling material and a translocation module having an amino acid sequence represented by SEQ ID NO:7; and (ii) a second fused construct comprising a polypeptide prey and a second labeling material;
(b) treating with a signaling material;
(c) inducing a translocation of the translocation module by treating with the signaling material that generates an external signal; and
(d) detecting a distribution of the first fused construct and the second fused construct in the cell.

3. The method of claim 1 or 2, wherein the first or the second labeling material is selected from the group consisting of GFP (Green Fluorescent Protein); EGFP (Enhanced Green Fluorescent Protein); RFP (Red Fluorescent Protein); mRFP (Monomeric Red Fluorescent Protein); DsRed (Discosoma sp. red fluorescent protein); CFP (Cyan Fluorescent Protein); CGFP (Cyan Green Fluorescent Protein); YFP (Yellow Fluorescent Protein); AzG (Azami Green), HcR (HcRed, Heteractis crispa red fluorescent protein), and BFP (Blue Fluorescent Protein).

4. The method of claim 1 or 2, wherein the first or the second fused construct further comprises a NLS(nuclear localization signal) sequence or a NES(nuclear export signal) sequence.

5. A method for detecting interactions of a polypeptide bait and a polypeptide prey comprising the steps of:
(a) preparing a cell comprising (i) a first fused construct comprising a polypeptide bait, a first labeling material which is EGFP (Enhanced Green Fluorescent Protein) or mRFP (Monomeric Red Fluorescent Protein) and a translocation module having an amino acid sequence represented by SEQ ID NO:7; and (ii) a second fused construct comprising a polypeptide prey and a second labeling material which is selected from the group consisting of EGFP (Enhanced Green Fluorescent Protein), mRFP (Monomeric Red Fluorescent Protein), AzG (Azami Green) and HcR (HcRed, Heteractis crispa red fluorescent protein);
(b) treating with a signaling material;
(c) inducing a translocation of the translocation module by treating with the signaling material that generates an external signal; and
(d) detecting a distribution of the first fused construct and the second fused construct in the cell,
wherein the first and the second labeling materials are different for distinction.

6. The method of claim 5, wherein the treatment with a signaling material is the treatment with 50 nM to 5 µM of PMA (Phorbol 12-myristate 13-acetate).

7. A method for screening materials which alter interactions of a polypeptide bait and a polypeptide prey comprising the steps of:
(a) preparing a cell comprising (i) a first fused construct comprising a polypeptide bait, a first labeling material and a translocation module having an amino acid sequence represented by SEQ ID NO:7; and (ii) a second fused construct comprising a polypeptide prey and a second labeling material;
(b) treating with a test agent; and
(c) inducing a translocation of the translocation module via an internal signaling mechanism or by treating with a signaling material that generates an external signal; and
(d) detecting a distribution of the first fused construct and the second fused construct in the cell, wherein the first labeling material is a fluorescent protein, wherein the second labeling material is a fluorescent protein, wherein the first and the second labeling materials are different for distinction.

8. The method of claim 7, wherein the alteration of the interactions is inhibition or promotion of the interaction.

9. The method of claim 7, wherein the method further comprises the step of treating with a signaling material.

10. A cell comprising (i) a first fused construct comprising a polypeptide bait, a first labeling material and a translocation module having an amino acid sequence represented by SEQ ID NO:7; and (ii) a second fused construct comprising a polypeptide prey and a second labeling material, wherein the first labeling material is a fluorescent protein, wherein the second labeling material is a fluorescent protein, wherein the first and the second labeling materials are different for distinction.

11. The cell of claim 10, wherein the cell was transformed with (i) an expression vector comprising a promoter and a nucleotide operably linked to the promoter and encoding a polypeptide bait, a first labeling material and a translocation module having an amino acid sequence represented by SEQ ID NO:7, and (ii) an expression vector comprising a promoter and a nucleotide operably linked to the promoter and encoding a polypeptide prey and a second labeling material.

12. The cell of claim 10, wherein the cell was co-transformed with (i) an expression vector comprising a promoter and a nucleotide operably linked to the promoter and encoding a polypeptide bait, a first labeling material which is EGFP (Enhanced Green Fluorescent Protein) or mRFP (Monomeric Red Fluorescent Protein) and a translocation module having an amino acid sequence represented by SEQ ID NO:7, and (ii) an expression vector comprising a promoter and a nucleotide operably linked to the promoter and encoding a polypeptide prey and a second labeling material which is selected from the group consisting of EGFP (Enhanced Green Fluorescent Protein), mRFP (Monomeric Red Fluorescent Protein), AzG (Azami Green), and HcR (HcRed, Heteractis crispa red fluorescent protein).

13. A kit for detecting interactions of a polypeptide bait and a polypeptide prey comprising the cell of any one of claim 10 to 12.

14. Use of the kit of claim 13 for detecting interactions of a polypeptide bait and a polypeptide prey and/or for screening materials which alter interactions of a polypeptide bait and a polypeptide prey.

## Patentansprüche

1. Verfahren zum Feststellen von Wechselwirkungen eines Köderpolypeptids und eines Beutepolypeptids, umfassend die folgenden Schritte:
a) Herstellen einer Zelle, umfassend: i) ein erstes Fusionskonstrukt, das ein Köderpolypeptid, ein erstes Markierungsmaterial und ein Translokationsmodul umfasst, das eine Aminosäuresequenz der SEQ ID Nr. 7 hat; und ii) ein zweites Fusionskonstrukt, das ein Beutepolypeptid und ein zweites Markierungsmaterial umfasst;
b) Induzieren einer Translokation des Translokationsmoduls über einen internen Signalisierungsmechanismus oder durch Behandlung mit einem Signalisierungsmaterial, das ein externes Signal erzeugt; und
c) Feststellen einer Verteilung des ersten Konstrukts und des zweiten Konstrukts in der Zelle, wobei das erste Markierungsmaterial ein fluoreszierendes Protein ist, wobei das zweite Markierungsmaterial ein fluoreszierendes Protein ist, wobei das erste und das zweite Markierungsmaterial zu Unterscheidungszwecken verschieden sind.

2. Verfahren gemäß Anspruch 1, zum Feststellen von Wechselwirkungen eines Köderpolypeptids und eines Beutepolypeptids, umfassend die folgenden Schritte:
a) Herstellen einer Zelle, umfassend: i) ein erstes Fusionskonstrukt, das ein Köderpolypeptid, ein erstes Markierungsmaterial und ein Translokationsmodul umfasst, das eine Aminosäuresequenz der SEQ ID Nr. 7 hat; und ii) ein zweites Fusionskonstrukt, das ein Beutepolypeptid und ein zweites Markierungsmaterial umfasst;
b) Behandeln mit einem Signalisierungsmaterial;
c) Induzieren einer Translokation des Translokationsmoduls durch Behandeln mit dem Signalisierungsmaterial, das ein externes Signal erzeugt; und
d) Feststellen einer Verteilung des ersten Fusionskonstrukts und des zweiten Fusionskonstrukts in der Zelle.

3. Verfahren gemäß Anspruch 1 oder 2, wobei das erste oder das zweite Markierungsmaterial aus der Gruppe ausgewählt ist, die aus GFP (grün fluoreszierendes Protein); EGFP (enhanced grün fluoreszierendes Protein); RFP (rot fluoreszierendes Protein); mRFP (monomeres rot fluoreszierendes Protein); DsRed (Discosoma sp. rot fluoreszierendes Protein); CFP (cyan fluoreszierendes Protein); CGFP (cyan-grün fluoreszierendes Protein); YFP (gelb fluoreszierendes Protein); AzG (Azami-Grün), HcR (HcRed, rot fluoreszierendes Protein aus Heteractis crispa) und BFP (blau fluoreszierendes Protein) besteht.

4. Verfahren gemäß Anspruch 1 oder 2, wobei das erste und das zweite Fusionskonstrukt ferner eine NLS(Zellkernlokalisierungssignal)-Sequenz oder eine NES(Zellkernexportsignal)-Sequenz umfasst.

5. Verfahren zum Feststellen von Wechselwirkungen eines Köderpolypeptids und eines Beutepolypeptids, umfassend die folgenden Schritte:
a) Herstellen einer Zelle, umfassend: i) ein erstes Fusionskonstrukt, das ein Köderpolypeptid, ein erstes Markierungsmaterial, das EGFP (enhanced grün fluoreszierendes Protein) oder mRFP (monomeres rot fluoreszierendes Protein) ist, und ein Translokationsmodul umfasst, das eine Aminosäuresequenz der SEQ ID Nr. 7 hat; und ii) ein zweites Fusionskonstrukt, das ein Beutepolypeptid und ein zweites Markierungsmaterial umfasst, das aus der Gruppe ausgewählt ist, die aus EGFP (enhanced grün fluoreszierendes Protein); mRFP (monomeres rot fluoreszierendes Protein); AzG (Azami-Grün) und HcR (HcRed, rot fluoreszierendes Protein aus Heteractis crispa) besteht;
b) Behandeln mit einem Signalisierungsmaterial;
c) Induzieren einer Translokation des Translokationsmoduls durch Behandeln mit dem Signalisierungsmaterial, das ein externes Signal erzeugt; und
d) Feststellen einer Verteilung des ersten Fusionskonstrukts und des zweiten Fusionskonstrukts in der Zelle, wobei das erste und das zweite Markierungsmaterial zu Unterscheidungszwecken verschieden sind.

6. Verfahren gemäß Anspruch 5, wobei die Behandlung mit einem Signalisierungsmaterial die Behandlung mit 50 nM bis 5 µM PMA (Phorbol-12-myristat-13-acetat) ist.

7. Verfahren zur Untersuchung von Materialien, die Wechselwirkungen eines Köderpolypeptids und eines Beutepolypeptids ändern, umfassend die folgenden Schritte:
a) Herstellen einer Zelle, umfassend: i) ein erstes Fusionskonstrukt, das ein Köderpolypeptid, ein erstes Markierungsmaterial und ein Translokationsmodul umfasst, das eine Aminosäuresequenz der SEQ ID Nr. 7 hat; und ii) ein zweites Fusionskonstrukt, das ein Beutepolypeptid und ein zweites Markierungsmaterial umfasst;
b) Behandeln mit einer Testsubstanz;
c) Induzieren einer Translokation des Translokationsmoduls über einen internen Signalisierungsmechanismus oder durch Behandlung mit einem Signalisierungsmaterial, das ein externes Signal erzeugt; und
d) Feststellen einer Verteilung des ersten Fusionskonstrukts und des zweiten Fusionskonstrukts in der Zelle, wobei das erste Markierungsmaterial ein fluoreszierendes Protein ist, wobei das zweite Markierungsmaterial ein fluoreszierendes Protein ist, wobei das erste und das zweite Markierungsmaterial zu Unterscheidungszwecken verschieden sind.

8. Verfahren gemäß Anspruch 7, wobei die Änderung der Wechselwirkung eine Hemmung oder Förderung der Wechselwirkung ist.

9. Verfahren gemäß Anspruch 7, wobei das Verfahren ferner den Schritt der Behandlung mit einem Signalisierungsmaterial umfasst.

10. Zelle, umfassend: i) ein erstes Fusionskonstrukt, das ein Köderpolypeptid, ein erstes Markierungsmaterial und ein Translokationsmodul umfasst, das eine Aminosäuresequenz der SEQ ID Nr. 7 hat; und ii) ein zweites Fusionskonstrukt, das ein Beutepolypeptid und ein zweites Markierungsmaterial umfasst, wobei das erste Markierungsmaterial ein fluoreszierendes Protein ist, wobei das zweite Markierungsmaterial ein fluoreszierendes Protein ist, wobei das erste und das zweite Markierungsmaterial zu Unterscheidungszwecken verschieden sind.

11. Zelle gemäß Anspruch 10, wobei die Zelle transformiert wurde mit: i) einem Expressionsvektor, der einen Promotor und ein Nukleotid, das operabel mit dem Promotor verbunden ist und ein Köderpolypeptid codiert, ein erstes Markierungsmaterial und ein Translokationsmodul umfasst, das eine Aminosäuresequenz der SEQ ID Nr. 7 hat, und ii) einem Expressionsvektor, der einen Promotor und ein Nukleotid, das operabel mit dem Promotor verbunden ist und ein Beutepolypeptid codiert, und ein zweites Markierungsmaterial umfasst.

12. Zelle gemäß Anspruch 10, wobei die Zelle cotransformiert war mit: i) einem Expressionsvektor, der einen Promotor und ein Nukleotid, das operabel mit dem Promotor verbunden ist und ein Köderpolypeptid codiert, ein erstes Markierungsmaterial, das EGFP (enhanced grün fluoreszierendes Protein) oder mRFP (monomeres rot fluoreszierendes Protein) ist, und ein Translokationsmodul umfasst, das eine Aminosäuresequenz der SEQ ID Nr. 7 hat, und ii) einem Expressionsvektor, der einen Promotor und ein Nukleotid, das operabel mit dem Promotor verbunden ist und ein Beutepolypeptid codiert, und ein zweites Markierungsmaterial umfasst, das aus der Gruppe ausgewählt ist, die aus EGFP (enhanced grün fluoreszierendes Protein), mRFP (monomeres rot fluoreszierendes Protein) AzG (Azami-Grün) und HcR (HcRed, rot fluoreszierendes Protein aus Heteractis crispa) besteht.

13. Testkit zum Feststellen von Wechselwirkungen eines Köderpolypeptids und eines Beutepolypeptids, umfassend die Zelle gemäß einem der Ansprüche 10 bis 12.

14. Verwendung eines Testkits gemäß Anspruch 13 zum Feststellen von Wechselwirkungen eines Köderpolypeptids und eines Beutepolypeptids und/oder zum Untersuchen von Materialien, die Wechselwirkungen eines Köderpolypeptids und eines Beutepolypeptids ändern.

## Revendications

1. Méthode de détection des interactions entre un appât polypeptidique et une proie polypeptidique comprenant les étapes de :
(a) préparer une cellule comprenant (i) une première construction fusionnée comprenant un appât polypeptidique, un premier matériau d'étiquetage et un module de translocation ayant une séquence d'acides aminés représentée par SEQ ID NO:7, et (ii) une deuxième construction fusionnée composée d'une proie polypeptidique et un deuxième matériau d'étiquetage,
(b) induire une translocation du module de translocation via un mécanisme interne de signalisation ou par traitement avec un matériau de signalisation qui génère un signal externe, et
(c) détecter une distribution de la première construction et de la deuxième dans la cellule, où le premier matériau d'étiquetage est une protéine fluorescente, où le deuxième matériau d'étiquetage est une protéine fluorescente, où le premier et le deuxième matériaux d'étiquetage sont différents pour pouvoir les distinguer.

2. Méthode selon la revendication 1 de détection des interactions entre un appât polypeptidique et une proie polypeptidique comprenant les étapes de :
(a) préparer une cellule comprenant (i) une première construction fusionnée comprenant un appât polypeptidique, un premier matériau d'étiquetage et un module de translocation ayant une séquence d'acides aminés représentée par la SEQ ID NO:7, et (ii) une deuxième construction fusionnée comprenant une proie polypeptidique et un deuxième matériau d'étiquetage,
(b) traiter avec un matériau de signalisation,
(c) induire une translocation du module de translocation par traitement avec le matériau de signalisation qui génère un signal externe, et
(d) détecter une distribution de la première construction fusionnée et de la deuxième construction fusionnée dans la cellule.

3. Méthode selon la revendication 1 ou 2, **caractérisée en ce que** le premier ou le deuxième matériau d'étiquetage est choisi dans le groupe composé de GFP (Protéine Fluorescente Verte), de EGFP (Protéine Fluorescente Verte Améliorée), de RFP (Protéine Fluorescente Rouge), de mRFP (Protéine Fluorescente Rouge monomérique), de DsRed (Protéine fluorescente rouge de *Discosoma sp.*), de CFP (Protéine Fluorescente Cyan), de CGFP (Protéine Fluorescente Verte Cyan), de YFP (Protéine Fluorescente Jaune), de AzG (Vert azami), de HcR (HcRed, protéine fluorescente rouge d'*Heteractis crispa*) et de BFP (Protéine Fluorescente Bleue).

4. Méthode selon la revendication 1 ou 2, **caractérisée en ce que** la première ou la deuxième construction fusionnées comprend en outre une séquence NLS (signal de localisation nucléaire) ou une séquence NES (signal d'exportation nucléaire).

5. Méthode de détection des interactions entre un appât polypeptidique et une proie polypeptidique comprenant les étapes de :
(a) préparer une cellule comprenant (i) une première construction fusionnée comprenant un appât polypeptidique, un premier matériau d'étiquetage qui est l'EGFP (Protéine Fluorescente Verte Améliorée) ou la mRFP (Protéine Fluorescente Rouge monomérique) et un module de translocation ayant une séquence d'acides aminés représentée par la SEQ ID NO:7, et (ii) une deuxième construction fusionnée comprenant une proie polypeptidique et un deuxième matériau d'étiquetage qui est choisi dans le groupe composé de EGFP (Protéine Fluorescente Verte Améliorée), de mRFP (Protéine Fluorescente Rouge monomérique), de AzG (Vert azami), et de HcR (HcRed, protéine fluorescente rouge d'*Heteractis crispa*),
(b) traiter avec un matériau de signalisation,
(c) induire une translocation du module de translocation par traitement avec le matériau de signalisation qui génère un signal externe, et
(d) détecter une distribution de la première construction fusionnée et de la deuxième construction fusionnée dans la cellule, où le premier et le deuxième matériaux d'étiquetage sont différents pour pouvoir les distinguer.

6. Méthode selon la revendication 5, **caractérisée en ce que** le traitement avec un matériau de signalisation est le traitement avec 50 nM à 5 µM de PMA (Phorbol 12-myristate 13-acétate).

7. Méthode pour trier les matériaux qui altèrent les interactions entre un appât polypeptidique et une proie polypeptidique comprenant les étapes de :
(a) préparer une cellule composée de (i) une première construction fusionnée comprenant un appât polypeptidique, un premier matériau d'étiquetage et un module de translocation ayant une séquence d'acides aminés représentée par la SEQ ID NO:7, et (ii) une deuxième construction fusionnée composé d'une proie polypeptidique et un deuxième matériau d'étiquetage,
(b) traiter avec un agent de test, et
(c) induire une translocation du module de translocation via un mécanisme de signalisation interne ou par traitement avec un matériau de signalisation qui génère un signal externe, et
(d) détecter une distribution de la première construction fusionnée et de la deuxième construction fusionnée dans la cellule, où le premier matériau d'étiquetage est une protéine fluorescente, où le deuxième matériau d'étiquetage est une protéine fluorescente, où le premier et le deuxième matériau d'étiquetage sont différents pour pouvoir les distinguer.

8. Méthode selon la revendication 7, **caractérisée en ce que** l'altération des interactions est l'inhibition ou la promotion de l'interaction.

9. Méthode selon la revendication 7, **caractérisée en ce que** la méthode comprend en outre l'étape de traitement avec un matériau de signalisation.

10. Cellule comprenant (i) une première construction fusionnée comprenant un appât polypeptidique, un premier matériau d'étiquetage et un module de translocation ayant une séquence d'acides aminés représentée par SEQ ID NO :7, et (ii) une deuxième construction fusionnée comprenant une proie polypeptidique et un deuxième matériau d'étiquetage, où le premier matériau d'étiquetage est une protéine fluorescente, où le deuxième matériau d'étiquetage est une protéine fluorescente, où le premier et le deuxième matériaux d'étiquetage sont différentes pour pouvoir les distinguer.

11. Cellule selon la revendication 10, **caractérisée en ce que** la cellule a été transformée avec (i) un vecteur d'expression comprenant un promoteur et un nucléotide lié de manière fonctionnelle au promoteur et codant pour un appât polypeptidique, un premier matériau d'étiquetage et un module de translocation ayant une séquence d'acides aminés représentée par SEQ ID NO :7, et (ii) un vecteur d'expression comprenant un promoteur et un nucléotide lié de manière fonctionnelle au promoteur et codant pour une proie polypeptidique et un deuxième matériau d'étiquetage.

12. Cellule selon la revendication 10, **caractérisée en ce que** la cellule a été co-transformée avec (i) un vecteur d'expression comprenant un promoteur et un nucléotide lié de manière fonctionnelle au promoteur et encodant un appât polypeptidique, un premier matériau d'étiquetage qui est l'EGFP (Protéine Fluorescente Verte Améliorée) ou la mRFP (Protéine Fluorescente Rouge monomérique) et un module de translocation ayant une séquence d'acides aminés représentée par SEQ ID NO :7, et (ii) un vecteur d'expression comprenant un promoteur et un nucléotide lié de manière fonctionnelle au promoteur et encodant un polypeptide proie et un deuxième matériau d'étiquetage qui est choisi dans le groupe composé de EGFP (Protéine Fluorescente Verte Améliorée), de mRFP (Protéine Fluorescente Rouge monomérique), de AzG (Vert Azami), et de HcR (HcRed, protéine fluorescente rouge d'*Heteractis crispa*).

13. Kit pour détecter les interactions entre un appât polypeptidique et une proie polypeptidique comprenant la cellule selon l'une quelconque des revendications 10 à 12.

14. Utilisation du kit selon la revendication 13 pour détecter les interactions entre un polypeptide appât et un polypeptide proie et/ou pour trier les matériaux qui altèrent les interactions entre un polypeptide appât et un polypeptide proie.


### Sequence Listing

<110> KOREA BASIC SCIENCE INSTITUTE
<120> METHOD FOR DETECTING INTERACTIONS BETWEEN TWO AND MORE BIOLOGICAL MACROMOLECULES
<130> P5226EP00
<150> KR 10-2008-0112354
   <151> 2008-11-12
<160> 99
<170> KopatentIn 1.71
<210> 1
   <211> 676
   <212> PRT
   <213> Homo sapiens
<400> 1
   Met Ala Pro Phe Leu Arg Ile Ala Phe Asn Ser Tyr Glu Leu Gly Ser
    1               5                  10                  15
   Leu Gln Ala Glu Asp Glu Ala Asn Gln Pro Phe Cys Ala Val Lys Met
               20                  25                  30
   Lys Glu Ala Leu Ser Thr Glu Arg Gly Lys Thr Leu Val Gln Lys Lys
           35                  40                  45
   Pro Thr Met Tyr Pro Glu Trp Lys Ser Thr Phe Asp Ala His Ile Tyr
       50                  55                  60
   Glu Gly Arg Val Ile Gln Ile Val Leu Met Arg Ala Ala Glu Glu Pro
   65                  70                  75                  80
   Val Ser Glu Val Thr Val Gly Val Ser Val Leu Ala Glu Arg Cys Lys
                   85                  90                  95
   Lys Asn Asn Gly Lys Ala Glu Phe Trp Leu Asp Leu Gln Pro Gln Ala
               100                 105                 110
   Lys Val Leu Met Ser Val Gln Tyr Phe Leu Glu Asp Val Asp Cys Lys
           115                 120                 125
   Gln Ser Met Arg Ser Glu Asp Glu Ala Lys Phe Pro Thr Met Asn Arg
       130                 135                 140
   Arg Gly Ala Ile Lys Gln Ala Lys Ile His Tyr Ile Lys Asn His Glu
   145                 150                 155                 160
   Phe Ile Ala Thr Phe Phe Gly Gln Pro Thr Phe Cys Ser Val Cys Lys
                   165                 170                 175
   Asp Phe Val Trp Gly Leu Asn Lys Gln Gly Tyr Lys Cys Arg Gln Cys
               180                 185                 190
   Asn Ala Ala Ile His Lys Lys Cys Ile Asp Lys Ile Ile Gly Arg Cys
           195                 200                 205
   Thr Gly Thr Ala Ala Asn Ser Arg Asp Thr Ile Phe Gln Lys Glu Arg
       210                 215                 220
   Phe Asn Ile Asp Met Pro His Arg Phe Lys Val His Asn Tyr Met Ser
   225                 230                 235                 240
   Pro Thr Phe Cys Asp His Cys Gly Ser Leu Leu Trp Gly Leu Val Lys
                   245                 250                 255
   Gln Gly Leu Lys Cys Glu Asp Cys Gly Met Asn Val His His Lys Cys
               260                 265                 270
   Arg Glu Lys Val Ala Asn Leu Cys Gly Ile Asn Gln Lys Leu Leu Ala
           275                 280                 285
   Glu Ala Leu Asn Gln Val Thr Gln Arg Ala Ser Arg Arg Ser Asp Ser
       290                 295                 300
   Ala Ser Ser Glu Pro Val Gly Ile Tyr Gln Gly Phe Glu Lys Lys Thr
   305                 310                 315                 320
   Gly Val Ala Gly Glu Asp Met Gln Asp Asn Ser Gly Thr Tyr Gly Lys
                   325                 330                 335
   Ile Trp Glu Gly Ser Ser Lys Cys Asn Ile Asn Asn Phe Ile Phe His
               340                 345                 350
   Lys Val Leu Gly Lys Gly Ser Phe Gly Lys Val Leu Leu Gly Glu Leu
           355                 360                 365
   Lys Gly Arg Gly Glu Tyr Phe Ala Ile Lys Ala Leu Lys Lys Asp Val
       370                 375                 380
   Val Leu Ile Asp Asp Asp Val Glu Cys Thr Met Val Glu Lys Arg Val
   385                 390                 395                 400
   Leu Thr Leu Ala Ala Glu Asn Pro Phe Leu Thr His Leu Ile Cys Thr
                   405                 410                 415
   Phe Gln Thr Lys Asp His Leu Phe Phe Val Met Glu Phe Leu Asn Gly
               420                 425                 430
   Gly Asp Leu Met Tyr His Ile Gln Asp Lys Gly Arg Phe Glu Leu Tyr
           435                 440                 445
   Arg Ala Thr Phe Tyr Ala Ala Glu Ile Met Cys Gly Leu Gln Phe Leu
       450                 455                 460
   His Ser Lys Gly Ile Ile Tyr Arg Asp Leu Lys Leu Asp Asn Val Leu
   465                 470                 475                 480
   Leu Asp Arg Asp Gly His Ile Lys Ile Ala Asp Phe Gly Met Cys Lys
                   485                 490                 495
   Glu Asn Ile Phe Gly Glu Ser Arg Ala Ser Thr Phe Cys Gly Thr Pro
               500                 505                 510
   Asp Tyr Ile Ala Pro Glu Ile Leu Gln Gly Leu Lys Tyr Thr Phe Ser
           515                 520                 525
   Val Asp Trp Trp Ser Phe Gly Val Leu Leu Tyr Glu Met Leu Ile Gly
       530                 535                 540
   Gln Ser Pro Phe His Gly Asp Asp Glu Asp Glu Leu Phe Glu Ser Ile
   545                 550                 555                 560
   Arg Val Asp Thr Pro His Tyr Pro Arg Trp Ile Thr Lys Glu Ser Lys
                   565                 570                 575
   Asp Ile Leu Glu Lys Leu Phe Glu Arg Glu Pro Thr Lys Arg Leu Gly
               580                 585                 590
   Val Thr Gly Asn Ile Lys Ile His Pro Phe Phe Lys Thr Ile Asn Trp
           595                 600                 605
   Thr Leu Leu Glu Lys Arg Arg Leu Glu Pro Pro Phe Arg Pro Lys Val
       610                 615                 620
   Lys Ser Pro Arg Asp Tyr Ser Asn Phe Asp Gln Glu Phe Leu Asn Glu
   625                 630                 635                 640
   Lys Ala Arg Leu Ser Tyr Ser Asp Lys Asn Leu Ile Asp Ser Met Asp
                   645                 650                 655
   Gln Ser Ala Phe Ala Gly Phe Ser Phe Val Asn Pro Lys Phe Glu His
               660                 665                 670
   Leu Leu Glu Asp
           675
<210> 2
   <211> 2028
   <212> DNA
   <213> Homo sapiens
<400> 2
   atggcgccgt tcctgcgcat cgccttcaac tcctatgagc tgggctccct gcaggccgag        60
   gacgaggcga accagccctt ctgtgccgtg aagatgaagg aggcgctcag cacagagcgt       120
   gggaaaacac tggtgcagaa gaagccgacc atgtatcctg agtggaagtc gacgttcgac       180
   gcccacatct atgaggggcg cgtcatccag attgtgctaa tgcgggcagc agaggagcca       240
   gtgtctgagg tgaccgtggg tgtgtcggtg ctggccgagc gctgcaagaa gaacaatggc       300
   aaggctgagt tctggctgga cctgcagcct caggccaagg tgttgatgtc tgttcagtat       360
   ttcctggagg acgtggattg caaacagtct atgcgcagtg aggacgaggc caagttccca       420
   acgatgaacc gccgcggagc catcaaacag gccaaaatcc actacatcaa gaaccatgag       480
   tttatcgcca ccttctttgg gcaacccacc ttctgttctg tgtgcaaaga ctttgtctgg     540
   ggcctcaaca agcaaggcta caaatgcagg caatgtaacg ctgccatcca caagaaatgc     600
   atcgacaaga tcatcggcag atgcactggc accgcggcca acagccggga cactatattc     660
   cagaaagaac gcttcaacat cgacatgccg caccgcttca aggttcacaa ctacatgagc     720
   cccaccttct gtgaccactg cggcagcctg ctctggggac tggtgaagca gggattaaag     780
   tgtgaagact gcggcatgaa tgtgcaccat aaatgccggg agaaggtggc caacctctgc     840
   ggcatcaacc agaagctttt ggctgaggcc ttgaaccaag tcacccagag agcctcccgg     900
   agatcagact cagcctcctc agagcctgtt gggatatatc agggtttcga gaagaagacc     960
   ggagttgctg gggaggacat gcaagacaac agtgggacct acggcaagat ctgggagggc    1020
   agcagcaagt gcaacatcaa caacttcatc ttccacaagg tcctgggcaa aggcagcttc    1080
   gggaaggtgc tgcttggaga gctgaagggc agaggagagt actttgccat caaggccctc    1140
   aagaaggatg tggtcctgat cgacgacgac gtggagtgca ccatggttga gaagcgggtg    1200
   ctgacacttg ccgcagagaa tccctttctc acccacctca tctgcacctt ccagaccaag    1260
   gaccacctgt tctttgtgat ggagttcctc aacggggggg acctgatgta ccacatccag    1320
   gacaaaggcc gctttgaact ctaccgtgcc acgttttatg ccgctgagat aatgtgtgga    1380
   ctgcagtttc tacacagcaa gggcatcatt tacagggacc tcaaactgga caatgtgctg    1440
   ttggaccggg atggccacat caagattgcc gactttggga tgtgcaaaga gaacatattc    1500
   ggggagagcc gggccagcac cttctgcggc acccctgact atatcgcccc tgagatccta    1560
   cagggcctga agtacacatt ctctgtggac tggtggtctt tcggggtcct tctgtacgag    1620
   atgctcattg gccagtcccc cttccatggt gatgatgagg atgaactctt cgagtccatc    1680
   cgtgtggaca cgccacatta tccccgctgg atcaccaagg agtccaagga catcctggag    1740
   aagctctttg aaagggaacc aaccaagagg ctgggagtga cgggaaacat caaaatccac    1800
   cccttcttca agaccataaa ctggactctg ctggaaaagc ggaggttgga gccacccttc    1860
   aggcccaaag tgaagtcacc cagagactac agtaactttg accaggagtt cctgaacgag    1920
   aaggcgcgcc tctcctacag cgacaagaac ctcatcgact ccatggacca gtctgcattc    1980
   gctggcttct cctttgtgaa ccccaaattc gagcacctcc tggaagat                 2028
<210> 3
   <211> 71
   <212> PRT
   <213> Artificial Sequence
<220>
   <223> TMA
<400> 3
   Met Lys Gln Ala Lys Ile His Tyr Ile Lys Asn His Glu Phe Ile Ala
   1               5                   10                  15
   Thr Phe Phe Gly Gln Pro Thr Phe Cys Ser Val Cys Lys Asp Phe Val
               20                  25                  30
   Trp Gly Leu Asn Lys Gln Gly Tyr Lys Cys Arg Gln Cys Asn Ala Ala
           35                  40                  45
   Ile His Lys Lys Cys Ile Asp Lys Ile Ile Gly Arg Cys Thr Gly Thr
       50                  55                  60
   Ala Ala Asn Ser Arg Asp Thr
   65                  70
<210> 4
   <211> 213
   <212> DNA
   <213> Artificial Sequence
<220>
   <223> TMA
<400> 4
   atgaaacagg ccaaaatcca ctacatcaag aaccatgagt ttatcgccac cttctttggg      60
   caacccacct tctgttctgt gtgcaaagac tttgtctggg gcctcaacaa gcaaggctac     120
   aaatgcaggc aatgtaacgc tgccatccac aagaaatgca tcgacaagat catcggcaga     180
   tgcactggca ccgcggccaa cagccgggac act                                  213
<210> 5
   <211> 71
   <212> PRT
   <213> Artificial Sequence
<220>
   <223> TMB
<400> 5
   Met Gln Lys Glu Arg Phe Asn Ile Asp Met Pro His Arg Phe Lys Val
   1               5                   10                  15
   His Asn Tyr Met Ser Pro Thr Phe Cys Asp His Cys Gly Ser Leu Leu
               20                  25                  30
   Trp Gly Leu Val Lys Gln Gly Leu Lys Cys Glu Asp Cys Gly Met Asn
   35 40 45
   Val His His Lys Cys Arg Glu Lys Val Ala Asn Leu Cys Gly Ile Asn
   50 55 60
   Gln Lys Leu Leu Ala Glu Ala
   65 70
<210> 6
   <211> 213
   <212> DNA
   <213> Artificial Sequence
<220>
   <223> TMB
<400> 6
   atgcagaaag aacgcttcaa catcgacatg ccgcaccgct tcaaggttca caactacatg 60
   agccccacct tctgtgacca ctgcggcagc ctgctctggg gactggtgaa gcagggatta 120
   aagtgtgaag actgcggcat gaatgtgcac cataaatgcc gggagaaggt ggccaacctc 180
   tgcggcatca accagaagct tttggctgag gcc 213
<210> 7
   <211> 676
   <212> PRT
   <213> Artificial Sequence
<220>
   <223> Protein kinase C mutant, TMD
<400> 7
   Met Ala Pro Phe Leu Arg Ile Ala Phe Asn Ser Tyr Glu Leu Gly Ser
   1 5 10 15
   Leu Gln Ala Glu Asp Glu Ala Asn Gln Pro Phe Cys Ala Val Lys Met
   20 25 30
   Lys Glu Ala Leu Ser Thr Glu Arg Gly Lys Thr Leu Val Gln Lys Lys
   35 40 45
   Pro Thr Met Tyr Pro Glu Trp Lys Ser Thr Phe Asp Ala His Ile Tyr
   50 55 60
   Glu Gly Arg Val Ile Gln Ile Val Leu Met Arg Ala Ala Glu Glu Pro
   65 70 75 80
   Val Ser Glu Val Thr Val Gly Val Ser Val Leu Ala Glu Arg Cys Lys
   85 90 95
   Lys Asn Asn Gly Lys Ala Glu Phe Trp Leu Asp Leu Gln Pro Gln Ala
               100                 105                 110
   Lys Val Leu Met Ser Val Gln Tyr Phe Leu Glu Asp Val Asp Cys Lys
           115                 120                 125
   Gln Ser Met Arg Ser Glu Asp Glu Ala Lys Phe Pro Thr Met Asn Arg
       130                 135                 140
   Arg Gly Ala Ile Lys Gln Ala Lys Ile His Tyr Ile Lys Asn His Glu
   145                 150                 155                 160
   Phe Ile Ala Thr Phe Phe Gly Gln Pro Thr Phe Cys Ser Val Cys Lys
                   165                 170                 175
   Asp Phe Val Trp Gly Leu Asn Lys Gln Gly Tyr Lys Cys Arg Gln Cys
               180                 185                 190
   Asn Ala Ala Ile His Lys Lys Cys Ile Asp Lys Ile Ile Gly Arg Cys
           195                 200                 205
   Thr Gly Thr Ala Ala Asn Ser Arg Asp Thr Ile Phe Gln Lys Glu Arg
       210                 215                 220
   Phe Asn Ile Asp Met Pro His Arg Phe Lys Val His Asn Tyr Met Ser
   225                 230                 235                 240
   Pro Thr Phe Cys Asp His Cys Gly Ser Leu Leu Trp Gly Leu Val Lys
                   245                 250                 255
   Gln Gly Leu Lys Cys Glu Asp Cys Gly Met Asn Val His His Lys Cys
               260                 265                 270
   Arg Glu Lys Val Ala Asn Leu Cys Gly Ile Asn Gln Lys Leu Leu Ala
           275                 280                 285
   Glu Ala Leu Asn Gln Val Thr Gln Arg Ala Ser Arg Arg Ser Asp Ser
       290                 295                 300
   Ala Ser Ser Glu Pro Val Gly Ile Phe Gln Gly Phe Glu Lys Lys Thr
   305                 310                 315                 320
   Gly Val Ala Gly Glu Asp Met Gln Asp Asn Ser Gly Thr Tyr Gly Lys
                   325                 330                 335
   Ile Trp Glu Gly Ser Ser Lys Cys Asn Ile Asn Asn Phe Ile Phe His
               340                 345                 350
   Lys Val Leu Gly Lys Gly Ser Phe Gly Lys Val Leu Leu Gly Glu Leu
           355                 360                 365
   Lys Gly Arg Gly Glu Tyr Phe Ala Ile Arg Ala Leu Lys Lys Asp Val
       370                 375                 380
   Val Leu Ile Asp Asp Asp Val Glu Cys Thr Met Val Glu Lys Arg Val
   385                 390                 395                 400
   Leu Thr Leu Ala Ala Glu Asn Pro Phe Leu Thr His Leu Ile Cys Thr
                   405                 410                 415
   Phe Gln Thr Lys Asp His Leu Phe Phe Val Met Glu Phe Leu Asn Gly
               420                 425                 430
   Gly Asp Leu Met Tyr His Ile Gln Asp Lys Gly Arg Phe Glu Leu Tyr
           435                 440                 445
   Arg Ala Thr Phe Tyr Ala Ala Glu Ile Met Cys Gly Leu Gln Phe Leu
       450                 455                 460
   His Ser Lys Gly Ile Ile Tyr Arg Asp Leu Lys Leu Asp Asn Val Leu
   465                 470                 475                 480
   Leu Asp Arg Asp Gly His Ile Lys Ile Ala Asp Phe Gly Met Cys Lys
                   485                 490                 495
   Glu Asn Ile Phe Gly Glu Ser Arg Ala Ser Thr Phe Cys Gly Thr Pro
               500                 505                 510
   Asp Tyr Ile Ala Pro Glu Ile Leu Gln Gly Leu Lys Tyr Thr Phe Ser
           515                 520                 525
   Val Asp Trp Trp Ser Phe Gly Val Leu Leu Tyr Glu Met Leu Ile Gly
       530                 535                 540
   Gln Ser Pro Phe His Gly Asp Asp Glu Asp Glu Leu Phe Glu Ser Ile
   545                 550                 555                 560
   Arg Val Asp Thr Pro His Tyr Pro Arg Trp Ile Thr Lys Glu Ser Lys
                   565                 570                 575
   Asp Ile Leu Glu Lys Leu Phe Glu Arg Glu Pro Thr Lys Arg Leu Gly
               580                 585                 590
   Val Thr Gly Asn Ile Lys Ile His Pro Phe Phe Lys Thr Ile Asn Trp
           595                 600                 605
   Thr Leu Leu Glu Lys Arg Arg Leu Glu Pro Pro Phe Arg Pro Lys Val
       610                 615                 620
   Lys Ser Pro Arg Asp Tyr Ser Asn Phe Asp Gln Glu Phe Leu Asn Glu
   625                 630                 635                 640
   Lys Ala Arg Leu Ser Tyr Ser Asp Lys Asn Leu Ile Asp Ser Met Asp
                   645                 650                 655
   Gln Ser Ala Phe Ala Gly Phe Ser Phe Val Asn Pro Lys Phe Glu His
               660                 665                 670
   Leu Leu Glu Asp
           675
<210> 8
   <211> 2028
   <212> DNA
   <213> Artificial Sequence
<220>
   <223> Protein kinase C mutant, TMD
<400> 8
   atggcgccgt tcctgcgcat cgccttcaac tcctatgagc tgggctccct gcaggccgag      60
   gacgaggcga accagccctt ctgtgccgtg aagatgaagg aggcgctcag cacagagcgt     120
   gggaaaacac tggtgcagaa gaagccgacc atgtatcctg agtggaagtc gacgttcgac     180
   gcccacatct atgaggggcg cgtcatccag attgtgctaa tgcgggcagc agaggagcca     240
   gtgtctgagg tgaccgtggg tgtgtcggtg ctggccgagc gctgcaagaa gaacaatggc     300
   aaggctgagt tctggctgga cctgcagcct caggccaagg tgttgatgtc tgttcagtat     360
   ttcctggagg acgtggattg caaacagtct atgcgcagtg aggacgaggc caagttccca     420
   acgatgaacc gccgcggagc catcaaacag gccaaaatcc actacatcaa gaaccatgag     480
   tttatcgcca ccttctttgg gcaacccacc ttctgttctg tgtgcaaaga ctttgtctgg     540
   ggcctcaaca agcaaggcta caaatgcagg caatgtaacg ctgccatcca caagaaatgc     600
   atcgacaaga tcatcggcag atgcactggc accgcggcca acagccggga cactatattc     660
   cagaaagaac gcttcaacat cgacatgccg caccgcttca aggttcacaa ctacatgagc     720
   cccaccttct gtgaccactg cggcagcctg ctctggggac tggtgaagca gggattaaag     780
   tgtgaagact gcggcatgaa tgtgcaccat aaatgccggg agaaggtggc caacctctgc     840
   ggcatcaacc agaagctttt ggctgaggcc ttgaaccaag tcacccagag agcctcccgg     900
   agatcagact cagcctcctc agagcctgtt gggatatttc agggtttcga gaagaagacc     960
   ggagttgctg gggaggacat gcaagacaac agtgggacct acggcaagat ctgggagggc    1020
   agcagcaagt gcaacatcaa caacttcatc ttccacaagg tcctgggcaa aggcagcttc    1080
   gggaaggtgc tgcttggaga gctgaagggc agaggagagt actttgccat cagggccctc    1140
   aagaaggatg tggtcctgat cgacgacgac gtggagtgca ccatggttga gaagcgggtg    1200
   ctgacacttg ccgcagagaa tccctttctc acccacctca tctgcacctt ccagaccaag    1260
   gaccacctgt tctttgtgat ggagttcctc aacggggggg acctgatgta ccacatccag    1320
   gacaaaggcc gctttgaact ctaccgtgcc acgttttatg ccgctgagat aatgtgtgga    1380
   ctgcagtttc tacacagcaa gggcatcatt tacagggacc tcaaactgga caatgtgctg    1440
   ttggaccggg atggccacat caagattgcc gactttggga tgtgcaaaga gaacatattc    1500
   ggggagagcc gggccagcac cttctgcggc acccctgact atatcgcccc tgagatccta    1560
   cagggcctga agtacacatt ctctgtggac tggtggtctt tcggggtcct tctgtacgag      1620
   atgctcattg gccagtcccc cttccatggt gatgatgagg atgaactctt cgagtccatc      1680
   cgtgtggaca cgccacatta tccccgctgg atcaccaagg agtccaagga catcctggag      1740
   aagctctttg aaagggaacc aaccaagagg ctgggagtga cgggaaacat caaaatccac      1800
   cccttcttca agaccataaa ctggactctg ctggaaaagc ggaggttgga gccacccttc      1860
   aggcccaaag tgaagtcacc cagagactac agtaactttg accaggagtt cctgaacgag      1920
   aaggcgcgcc tctcctacag cgacaagaac ctcatcgact ccatggacca gtctgcattc      1980
   gctggcttct cctttgtgaa ccccaaattc gagcacctcc tggaagat                    2028
<210> 9
   <211> 239
   <212> PRT
   <213> Artificial Sequence
<220>
   <223> EGFP
<400> 9
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 10
   <211> 716
   <212> DNA
   <213> Artificial Sequence
<220>
   <223> EGFP
<400> 10
   tggtgagcaa gggcgaggag ctgttcaccg gggtggtgcc catcctggtc gagctggacg      60
   gcgacgtaaa cggccacaag ttcagcgtgt ccggcgaggg cgagggcgat gccacctacg     120
   gcaagctgac cctgaagttc atctgcacca ccggcaagct gcccgtgccc tggcccaccc     180
   tcgtgaccac cctgacctac ggcgtgcagt gcttcagccg ctaccccgac cacatgaagc     240
   agcacgactt cttcaagtcc gccatgcccg aaggctacgt ccaggagcgc accatcttct     300
   tcaaggacga cggcaactac aagacccgcg ccgaggtgaa gttcgagggc gacaccctgg     360
   tgaaccgcat cgagctgaag ggcatcgact tcaaggagga cggcaacatc ctggggcaca     420
   agctggagta caactacaac agccacaacg tctatatcat ggccgacaag cagaagaacg     480
   gcatcaaggt gaacttcaag atccgccaca acatcgagga cggcagcgtg cagctcgccg     540
   accactacca gcagaacacc cccatcggcg acggccccgt gctgctgccc gacaaccact     600
   acctgagcac ccagtccgcc ctgagcaaag accccaacga gaagcgcgat cacatggtcc     660
   tgctggagtt cgtgaccgcc gccgggatca ctctcggcat ggacgagctg tacaag         716
<210> 11
   <211> 225
   <212> PRT
   <213> Artificial Sequence
<220>
   <223> mRFP
<400> 11
   Met Ala Ser Ser Glu Asp Val Ile Lys Glu Phe Met Arg Phe Lys Val
   1               5                   10                  15
   Arg Met Glu Gly Ser Val Asn Gly His Glu Phe Glu Ile Glu Gly Glu
               20                  25                  30
   Gly Glu Gly Arg Pro Tyr Glu Gly Thr Gln Thr Ala Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Ala Tyr Val Lys His Pro Ala Asp Ile Pro
   65                  70                  75                  80
   Asp Tyr Leu Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Glu Phe Ile Tyr Lys Val Lys Leu Arg Gly Thr Asn
           115                 120                 125
   Phe Pro Ser Asp Gly Pro Val Met Gln Lys Lys Thr Met Gly Trp Glu
       130                 135                 140
   Ala Ser Thr Glu Arg Met Tyr Pro Glu Asp Gly Ala Leu Lys Gly Glu
   145                 150                 155                 160
   Ile Lys Met Arg Leu Lys Leu Lys Asp Gly Gly His Tyr Asp Ala Glu
                   165                 170                 175
   Val Lys Thr Thr Tyr Met Ala Lys Lys Pro Val Gln Leu Pro Gly Ala
               180                 185                 190
   Tyr Lys Thr Asp Ile Lys Leu Asp Ile Thr Ser His Asn Glu Asp Tyr
           195                 200                 205
   Thr Ile Val Glu Gln Tyr Glu Arg Ala Glu Gly Arg His Ser Thr Gly
       210                 215                 220
   Ala
   225
<210> 12
   <211> 674
   <212> DNA
   <213> Artificial Sequence
<220>
   <223> mRFP
<400> 12
   tggcctcctc cgaggacgtc atcaaggagt tcatgcgctt caaggtgcgc atggagggct      60
   ccgtgaacgg ccacgagttc gagatcgagg gcgagggcga gggccgcccc tacgagggca     120
   cccagaccgc caagctgaag gtgaccaagg gcggccccct gcccttcgcc tgggacatcc     180
   tgtcccctca gttccagtac ggctccaagg cctacgtgaa gcaccccgcc gacatccccg     240
   actacttgaa gctgtccttc cccgagggct tcaagtggga gcgcgtgatg aacttcgagg     300
   acggcggcgt ggtgaccgtg acccaggact cctccctgca ggacggcgag ttcatctaca     360
   aggtgaagct gcgcggcacc aacttcccct ccgacggccc cgtaatgcag aagaagacca     420
   tgggctggga ggcctccacc gagcggatgt accccgagga cggcgccctg aagggcgaga     480
   tcaagatgag gctgaagctg aaggacggcg gccactacga cgccgaggtc aagaccacct     540
   acatggccaa gaagcccgtg cagctgcccg gcgcctacaa gaccgacatc aagctggaca     600
   tcacctccca caacgaggac tacaccatcg tggaacagta cgagcgcgcc gagggccgcc     660
   actccaccgg cgcc                                                       674
<210> 13
   <211> 229
   <212> PRT
   <213> Artificial Sequence
<220>
   <223> AzG
<400> 13
   Met Asp Pro Met Val Ser Val Ile Lys Pro Glu Met Lys Ile Lys Leu
   1               5                   10                  15
   Cys Met Arg Gly Thr Val Asn Gly His Asn Phe Val Ile Glu Gly Glu
               20                  25                  30
   Gly Lys Gly Asn Pro Tyr Glu Gly Thr Gln Ile Leu Asp Leu Asn Val
           35                  40                  45
   Thr Glu Gly Ala Pro Leu Pro Phe Ala Tyr Asp Ile Leu Thr Thr Val
       50                  55                  60
   Phe Gln Tyr Gly Asn Arg Ala Phe Thr Lys Tyr Pro Ala Asp Ile Gln
   65                  70                  75                  80
   Asp Tyr Phe Lys Gln Thr Phe Pro Glu Gly Tyr His Trp Glu Arg Ser
                   85                  90                  95
   Met Thr Tyr Glu Asp Gln Gly Ile Cys Thr Ala Thr Ser Asn Ile Ser
               100                 105                 110
   Met Arg Gly Asp Cys Phe Phe Tyr Asp Ile Arg Phe Asp Gly Thr Asn
           115                 120                 125
   Phe Pro Pro Asn Gly Pro Val Met Gln Lys Lys Thr Leu Lys Trp Glu
       130                 135                 140
   Pro Ser Thr Glu Lys Met Tyr Val Glu Asp Gly Val Leu Lys Gly Asp
   145                 150                 155                 160
   Val Asn Met Arg Leu Leu Leu Glu Gly Gly Gly His Tyr Arg Cys Asp
                   165                 170                 175
   Phe Lys Thr Thr Tyr Lys Ala Lys Lys Glu Val Arg Leu Pro Asp Ala
               180                 185                 190
   His Lys Ile Asp His Arg Ile Glu Ile Leu Lys His Asp Lys Asp Tyr
           195                 200                 205
   Asn Lys Val Lys Leu Tyr Glu Asn Ala Val Ala Arg Tyr Ser Met Leu
       210                 215                 220
   Pro Ser Gln Ala Lys
   225
<210> 14
   <211> 687
   <212> DNA
   <213> Artificial Sequence
<220>
   <223> AzG
<400> 14
   atggacccca tggtgagtgt gattaaacca gagatgaaga tcaagctgtg tatgagaggc      60
   actgtaaacg ggcataattt cgtgattgaa ggagaaggaa aaggaaatcc ttacgaggga     120
   acgcagattt tagacctgaa cgtcactgaa ggcgcacctc tgcctttcgc ttacgatatc     180
   ttgacaacag tgttccagta cggcaacagg gcattcacca agtacccagc agatattcag     240
   gactatttca agcagacttt tcctgagggg tatcactggg aaagaagcat gacttatgaa     300
   gaccagggca tttgcaccgc cacaagcaac ataagcatga ggggcgactg ttttttctat      360
   gacattcgtt ttgatggcac caactttcct cccaatggtc cggttatgca gaagaagact     420
   cttaaatggg agccatccac tgagaaaatg tacgtagagg atggagtgct gaagggtgat     480
   gttaacatgc gcctgttgct tgaaggaggt ggccattatc gatgtgattt caaaactact     540
   tacaaagcaa agaaggaggt ccgtttgcca gacgcgcaca aaattgacca ccgcattgag     600
   attttgaagc atgacaaaga ttacaacaag gtcaagctct atgagaatgc cgttgctcgc     660
   tattctatgc tgccgagtca ggccaag                                         687
<210> 15
   <211> 228
   <212> PRT
   <213> Artificial Sequence
<220>
   <223> HcR
<400> 15
   Met Val Ser Gly Leu Leu Lys Glu Ser Met Arg Ile Lys Met Tyr Met
   1               5                   10                  15
   Glu Gly Thr Val Asn Gly His Tyr Phe Lys Cys Glu Gly Glu Gly Asp
               20                  25                  30
   Gly Asn Pro Phe Ala Gly Thr Gln Ser Met Arg Ile His Val Thr Glu
           35                  40                  45
   Gly Ala Pro Leu Pro Phe Ala Phe Asp Ile Leu Ala Pro Cys Cys Glu
       50                  55                  60
   Tyr Gly Ser Arg Thr Phe Val His His Thr Ala Glu Ile Pro Asp Phe
   65                  70                  75                  80
   Phe Lys Gln Ser Phe Pro Glu Gly Phe Thr Trp Glu Arg Thr Thr Thr
                   85                  90                  95
   Tyr Glu Asp Gly Gly Ile Leu Thr Ala His Gln Asp Thr Ser Leu Glu
               100                 105                 110
   Gly Asn Cys Leu Ile Tyr Lys Val Lys Val His Gly Thr Asn Phe Pro
           115                 120                 125
   Ala Asp Gly Pro Val Met Lys Asn Lys Ser Gly Gly Trp Glu Pro Ser
       130                 135                 140
   Thr Glu Val Val Tyr Pro Glu Asn Gly Val Leu Cys Gly Arg Asn Val
   145                 150                 155                 160
   Met Ala Leu Lys Val Gly Asp Arg His Leu Ile Cys His His Tyr Thr
                   165                 170                 175
   Ser Tyr Arg Ser Lys Lys Ala Val Arg Ala Leu Thr Met Pro Gly Phe
               180                 185                 190
   His Phe Thr Asp Ile Arg Leu Gln Met Leu Arg Lys Lys Lys Asp Glu
           195                 200                 205
   Tyr Phe Glu Leu Tyr Glu Ala Ser Val Ala Arg Tyr Ser Asp Leu Pro
       210                 215                 220
   Glu Lys Ala Asn
   225
<210> 16
   <211> 684
   <212> DNA
   <213> Artificial Sequence
<220>
   <223> HcR
<400> 16
   atggtgagcg gcctgctgaa ggagagtatg cgcatcaaga tgtacatgga gggcaccgtg        60
   aacggccact acttcaagtg cgagggcgag ggcgacggca accccttcgc cggcacccag       120
   agcatgagaa tccacgtgac cgagggcgcc cccctgccct tcgccttcga catcctggcc     180
   ccctgctgcg agtacggcag caggaccttc gtgcaccaca ccgccgagat ccccgacttc     240
   ttcaagcaga gcttccccga gggcttcacc tgggagagaa ccaccaccta cgaggacggc     300
   ggcatcctga ccgcccacca ggacaccagc ctggagggca actgcctgat ctacaaggtg     360
   aaggtgcacg gcaccaactt ccccgccgac ggccccgtga tgaagaacaa gagcggcggc     420
   tgggagccca gcaccgaggt ggtgtacccc gagaacggcg tgctgtgcgg ccggaacgtg     480
   atggccctga aggtgggcga ccggcacctg atctgccacc actacaccag ctaccggagc     540
   aagaaggccg tgcgcgccct gaccatgccc ggcttccact tcaccgacat ccggctccag     600
   atgctgcgga agaagaagga cgagtacttc gagctgtacg aggccagcgt ggcccggtac     660
   agcgacctgc ccgagaaggc caac                                            684
<210> 17
   <211> 23
   <212> PRT
   <213> Artificial Sequence
<220>
   <223> NLS
<400> 17
   Gly Ser Gly Asp Glu Val Glu Gly Val Glu Glu Val Ala Lys Lys Lys
   1               5                   10                  15
   Ser Lys Lys Glu Lys Asp Lys
               20
<210> 18
   <211> 69
   <212> DNA
   <213> Artificial Sequence
<220>
   <223> NLS
<400> 18
   ggctctggtg atgaagtcga aggagtggaa gaagtagcta agaagaagag taaaaaggaa 60
   aaggataaa 69
<210> 19
   <211> 18
   <212> PRT
   <213> Artificial Sequence
<220>
   <223> NES
<400> 19
   Asp Gln Arg Val Ile Ile Lys Leu Asn Ala His Val Gly Asn Ile Ser
   1 5 10 15
   Leu Val
<210> 20
   <211> 54
   <212> DNA
   <213> Artificial Sequence
<220>
   <223> NES
<400> 20
   gaccagcgcg tcatcatcaa gctgaacgcc catgtgggaa acatttccct ggtg 54
<210> 21
   <211> 4727
   <212> DNA
   <213> Artificial Sequence
<220>
   <223> pEGFP-C3 vector
<400> 21
   tagttattaa tagtaatcaa ttacggggtc attagttcat agcccatata tggagttccg 60
   cgttacataa cttacggtaa atggcccgcc tggctgaccg cccaacgacc cccgcccatt 120
   gacgtcaata atgacgtatg ttcccatagt aacgccaata gggactttcc attgacgtca 180
   atgggtggag tatttacggt aaactgccca cttggcagta catcaagtgt atcatatgcc 240
   aagtacgccc cctattgacg tcaatgacgg taaatggccc gcctggcatt atgcccagta 300
   catgacctta tgggactttc ctacttggca gtacatctac gtattagtca tcgctattac     360
   catggtgatg cggttttggc agtacatcaa tgggcgtgga tagcggtttg actcacgggg     420
   atttccaagt ctccacccca ttgacgtcaa tgggagtttg ttttggcacc aaaatcaacg     480
   ggactttcca aaatgtcgta acaactccgc cccattgacg caaatgggcg gtaggcgtgt     540
   acggtgggag gtctatataa gcagagctgg tttagtgaac cgtcagatcc gctagcgcta     600
   ccggtcgcca ccatggtgag caagggcgag gagctgttca ccggggtggt gcccatcctg     660
   gtcgagctgg acggcgacgt aaacggccac aagttcagcg tgtccggcga gggcgagggc     720
   gatgccacct acggcaagct gaccctgaag ttcatctgca ccaccggcaa gctgcccgtg     780
   ccctggccca ccctcgtgac caccctgacc tacggcgtgc agtgcttcag ccgctacccc     840
   gaccacatga agcagcacga cttcttcaag tccgccatgc ccgaaggcta cgtccaggag     900
   cgcaccatct tcttcaagga cgacggcaac tacaagaccc gcgccgaggt gaagttcgag     960
   ggcgacaccc tggtgaaccg catcgagctg aagggcatcg acttcaagga ggacggcaac    1020
   atcctggggc acaagctgga gtacaactac aacagccaca acgtctatat catggccgac    1080
   aagcagaaga acggcatcaa ggtgaacttc aagatccgcc acaacatcga ggacggcagc    1140
   gtgcagctcg ccgaccacta ccagcagaac acccccatcg gcgacggccc cgtgctgctg    1200
   cccgacaacc actacctgag cacccagtcc gccctgagca aagaccccaa cgagaagcgc    1260
   gatcacatgg tcctgctgga gttcgtgacc gccgccggga tcactctcgg catggacgag    1320
   ctgtacaagt actcagatct cgagctcaag cttcgaattc tgcagtcgac ggtaccgcgg    1380
   gcccgggatc caccggatct agataactga tcataatcag ccataccaca tttgtagagg    1440
   ttttacttgc tttaaaaaac ctcccacacc tccccctgaa cctgaaacat aaaatgaatg     1500
   caattgttgt tgttaacttg tttattgcag cttataatgg ttacaaataa agcaatagca    1560
   tcacaaattt cacaaataaa gcattttttt cactgcattc tagttgtggt ttgtccaaac    1620
   tcatcaatgt atcttaacgc gtaaattgta agcgttaata ttttgttaaa attcgcgtta    1680
   aatttttgtt aaatcagctc attttttaac caataggccg aaatcggcaa aatcccttat    1740
   aaatcaaaag aatagaccga gatagggttg agtgttgttc cagtttggaa caagagtcca    1800
   ctattaaaga acgtggactc caacgtcaaa gggcgaaaaa ccgtctatca gggcgatggc    1860
   ccactacgtg aaccatcacc ctaatcaagt tttttggggt cgaggtgccg taaagcacta    1920
   aatcggaacc ctaaagggag cccccgattt agagcttgac ggggaaagcc ggcgaacgtg    1980
   gcgagaaagg aagggaagaa agcgaaagga gcgggcgcta gggcgctggc aagtgtagcg    2040
   gtcacgctgc gcgtaaccac cacacccgcc gcgcttaatg cgccgctaca gggcgcgtca 2100
   ggtggcactt ttcggggaaa tgtgcgcgga acccctattt gtttattttt ctaaatacat 2160
   tcaaatatgt atccgctcat gagacaataa ccctgataaa tgcttcaata atattgaaaa 2220
   aggaagagtc ctgaggcgga aagaaccagc tgtggaatgt gtgtcagtta gggtgtggaa 2280
   agtccccagg ctccccagca ggcagaagta tgcaaagcat gcatctcaat tagtcagcaa 2340
   ccaggtgtgg aaagtcccca ggctccccag caggcagaag tatgcaaagc atgcatctca 2400
   attagtcagc aaccatagtc ccgcccctaa ctccgcccat cccgccccta actccgccca 2460
   gttccgccca ttctccgccc catggctgac taattttttt tatttatgca gaggccgagg 2520
   ccgcctcggc ctctgagcta ttccagaagt agtgaggagg cttttttgga ggcctaggct 2580
   tttgcaaaga tcgatcaaga gacaggatga ggatcgtttc gcatgattga acaagatgga 2640
   ttgcacgcag gttctccggc cgcttgggtg gagaggctat tcggctatga ctgggcacaa 2700
   cagacaatcg gctgctctga tgccgccgtg ttccggctgt cagcgcaggg gcgcccggtt 2760
   ctttttgtca agaccgacct gtccggtgcc ctgaatgaac tgcaagacga ggcagcgcgg 2820
   ctatcgtggc tggccacgac gggcgttcct tgcgcagctg tgctcgacgt tgtcactgaa 2880
   gcgggaaggg actggctgct attgggcgaa gtgccggggc aggatctcct gtcatctcac 2940
   cttgctcctg ccgagaaagt atccatcatg gctgatgcaa tgcggcggct gcatacgctt 3000
   gatccggcta cctgcccatt cgaccaccaa gcgaaacatc gcatcgagcg agcacgtact 3060
   cggatggaag ccggtcttgt cgatcaggat gatctggacg aagagcatca ggggctcgcg 3120
   ccagccgaac tgttcgccag gctcaaggcg agcatgcccg acggcgagga tctcgtcgtg 3180
   acccatggcg atgcctgctt gccgaatatc atggtggaaa atggccgctt ttctggattc 3240
   atcgactgtg gccggctggg tgtggcggac cgctatcagg acatagcgtt ggctacccgt 3300
   gatattgctg aagagcttgg cggcgaatgg gctgaccgct tcctcgtgct ttacggtatc 3360
   gccgctcccg attcgcagcg catcgccttc tatcgccttc ttgacgagtt cttctgagcg 3420
   ggactctggg gttcgaaatg accgaccaag cgacgcccaa cctgccatca cgagatttcg 3480
   attccaccgc cgccttctat gaaaggttgg gcttcggaat cgttttccgg gacgccggct 3540
   ggatgatcct ccagcgcggg gatctcatgc tggagttctt cgcccaccct agggggaggc 3600
   taactgaaac acggaaggag acaataccgg aaggaacccg cgctatgacg gcaataaaaa 3660
   gacagaataa aacgcacggt gttgggtcgt ttgttcataa acgcggggtt cggtcccagg 3720
   gctggcactc tgtcgatacc ccaccgagac ccattggggc caatacgccc gcgtttcttc 3780
   cctttccccc accccacccc ccaagttcgg gtgaaggccc agggctcgca gccaacgtcg 3840
   gggcggcagg ccctgccata gcctcaggtt actcatatat actttagatt gatttaaaac 3900
   ttcattttta atttaaaagg atctaggtga agatcctttt tgataatctc atgaccaaaa 3960
   tcccttaacg tgagttttcg ttccactgag cgtcagaccc cgtagaaaag atcaaaggat 4020
   cttcttgaga tccttttttt ctgcgcgtaa tctgctgctt gcaaacaaaa aaaccaccgc 4080
   taccagcggt ggtttgtttg ccggatcaag agctaccaac tctttttccg aaggtaactg 4140
   gcttcagcag agcgcagata ccaaatactg tccttctagt gtagccgtag ttaggccacc 4200
   acttcaagaa ctctgtagca ccgcctacat acctcgctct gctaatcctg ttaccagtgg 4260
   ctgctgccag tggcgataag tcgtgtctta ccgggttgga ctcaagacga tagttaccgg 4320
   ataaggcgca gcggtcgggc tgaacggggg gttcgtgcac acagcccagc ttggagcgaa 4380
   cgacctacac cgaactgaga tacctacagc gtgagctatg agaaagcgcc acgcttcccg 4440
   aagggagaaa ggcggacagg tatccggtaa gcggcagggt cggaacagga gagcgcacga 4500
   gggagcttcc agggggaaac gcctggtatc tttatagtcc tgtcgggttt cgccacctct 4560
   gacttgagcg tcgatttttg tgatgctcgt caggggggcg gagcctatgg aaaaacgcca 4620
   gcaacgcggc ctttttacgg ttcctggcct tttgctggcc ttttgctcac atgttctttc 4680
   ctgcgttatc ccctgattct gtggataacc gtattaccgc catgcat 4727
<210> 22
   <211> 4685
   <212> DNA
   <213> Artificial Sequence
<220>
   <223> pmRFP-C3 vector
<400> 22
   tagttattaa tagtaatcaa ttacggggtc attagttcat agcccatata tggagttccg 60
   cgttacataa cttacggtaa atggcccgcc tggctgaccg cccaacgacc cccgcccatt 120
   gacgtcaata atgacgtatg ttcccatagt aacgccaata gggactttcc attgacgtca 180
   atgggtggag tatttacggt aaactgccca cttggcagta catcaagtgt atcatatgcc 240
   aagtacgccc cctattgacg tcaatgacgg taaatggccc gcctggcatt atgcccagta 300
   catgacctta tgggactttc ctacttggca gtacatctac gtattagtca tcgctattac 360
   catggtgatg cggttttggc agtacatcaa tgggcgtgga tagcggtttg actcacgggg 420
   atttccaagt ctccacccca ttgacgtcaa tgggagtttg ttttggcacc aaaatcaacg 480
   ggactttcca aaatgtcgta acaactccgc cccattgacg caaatgggcg gtaggcgtgt 540
   acggtgggag gtctatataa gcagagctgg tttagtgaac cgtcagatcc gctagcgcta 600
   ccggtcgcca ccatggcctc ctccgaggac gtcatcaagg agttcatgcg cttcaaggtg 660
   cgcatggagg gctccgtgaa cggccacgag ttcgagatcg agggcgaggg cgagggccgc 720
   ccctacgagg gcacccagac cgccaagctg aaggtgacca agggcggccc cctgcccttc 780
   gcctgggaca tcctgtcccc tcagttccag tacggctcca aggcctacgt gaagcacccc 840
   gccgacatcc ccgactactt gaagctgtcc ttccccgagg gcttcaagtg ggagcgcgtg 900
   atgaacttcg aggacggcgg cgtggtgacc gtgacccagg actcctccct gcaggacggc 960
   gagttcatct acaaggtgaa gctgcgcggc accaacttcc cctccgacgg ccccgtaatg 1020
   cagaagaaga ccatgggctg ggaggcctcc accgagcgga tgtaccccga ggacggcgcc 1080
   ctgaagggcg agatcaagat gaggctgaag ctgaaggacg gcggccacta cgacgccgag 1140
   gtcaagacca cctacatggc caagaagccc gtgcagctgc ccggcgccta caagaccgac 1200
   atcaagctgg acatcacctc ccacaacgag gactacacca tcgtggaaca gtacgagcgc 1260
   gccgagggcc gccactccac cggcgccctg tcagatctcg agctcaagct tcgaattctg 1320
   cagtcgacgg taccgcgggc ccgggatcca ccggatctag ataactgatc ataatcagcc 1380
   ataccacatt tgtagaggtt ttacttgctt taaaaaacct cccacacctc cccctgaacc 1440
   tgaaacataa aatgaatgca attgttgttg ttaacttgtt tattgcagct tataatggtt 1500
   acaaataaag caatagcatc acaaatttca caaataaagc atttttttca ctgcattcta 1560
   gttgtggttt gtccaaactc atcaatgtat cttaacgcgt aaattgtaag cgttaatatt 1620
   ttgttaaaat tcgcgttaaa tttttgttaa atcagctcat tttttaacca ataggccgaa 1680
   atcggcaaaa tcccttataa atcaaaagaa tagaccgaga tagggttgag tgttgttcca 1740
   gtttggaaca agagtccact attaaagaac gtggactcca acgtcaaagg gcgaaaaacc 1800
   gtctatcagg gcgatggccc actacgtgaa ccatcaccct aatcaagttt tttggggtcg 1860
   aggtgccgta aagcactaaa tcggaaccct aaagggagcc cccgatttag agcttgacgg 1920
   ggaaagccgg cgaacgtggc gagaaaggaa gggaagaaag cgaaaggagc gggcgctagg 1980
   gcgctggcaa gtgtagcggt cacgctgcgc gtaaccacca cacccgccgc gcttaatgcg 2040
   ccgctacagg gcgcgtcagg tggcactttt cggggaaatg tgcgcggaac ccctatttgt 2100
   ttatttttct aaatacattc aaatatgtat ccgctcatga gacaataacc ctgataaatg 2160
   cttcaataat attgaaaaag gaagagtcct gaggcggaaa gaaccagctg tggaatgtgt 2220
   gtcagttagg gtgtggaaag tccccaggct ccccagcagg cagaagtatg caaagcatgc 2280
   atctcaatta gtcagcaacc aggtgtggaa agtccccagg ctccccagca ggcagaagta 2340
   tgcaaagcat gcatctcaat tagtcagcaa ccatagtccc gcccctaact ccgcccatcc 2400
   cgcccctaac tccgcccagt tccgcccatt ctccgcccca tggctgacta atttttttta 2460
   tttatgcaga ggccgaggcc gcctcggcct ctgagctatt ccagaagtag tgaggaggct 2520
   tttttggagg cctaggcttt tgcaaagatc gatcaagaga caggatgagg atcgtttcgc 2580
   atgattgaac aagatggatt gcacgcaggt tctccggccg cttgggtgga gaggctattc 2640
   ggctatgact gggcacaaca gacaatcggc tgctctgatg ccgccgtgtt ccggctgtca 2700
   gcgcaggggc gcccggttct ttttgtcaag accgacctgt ccggtgccct gaatgaactg 2760
   caagacgagg cagcgcggct atcgtggctg gccacgacgg gcgttccttg cgcagctgtg 2820
   ctcgacgttg tcactgaagc gggaagggac tggctgctat tgggcgaagt gccggggcag 2880
   gatctcctgt catctcacct tgctcctgcc gagaaagtat ccatcatggc tgatgcaatg 2940
   cggcggctgc atacgcttga tccggctacc tgcccattcg accaccaagc gaaacatcgc 3000
   atcgagcgag cacgtactcg gatggaagcc ggtcttgtcg atcaggatga tctggacgaa 3060
   gagcatcagg ggctcgcgcc agccgaactg ttcgccaggc tcaaggcgag catgcccgac 3120
   ggcgaggatc tcgtcgtgac ccatggcgat gcctgcttgc cgaatatcat ggtggaaaat 3180
   ggccgctttt ctggattcat cgactgtggc cggctgggtg tggcggaccg ctatcaggac 3240
   atagcgttgg ctacccgtga tattgctgaa gagcttggcg gcgaatgggc tgaccgcttc 3300
   ctcgtgcttt acggtatcgc cgctcccgat tcgcagcgca tcgccttcta tcgccttctt 3360
   gacgagttct tctgagcggg actctggggt tcgaaatgac cgaccaagcg acgcccaacc 3420
   tgccatcacg agatttcgat tccaccgccg ccttctatga aaggttgggc ttcggaatcg 3480
   ttttccggga cgccggctgg atgatcctcc agcgcgggga tctcatgctg gagttcttcg 3540
   cccaccctag ggggaggcta actgaaacac ggaaggagac aataccggaa ggaacccgcg 3600
   ctatgacggc aataaaaaga cagaataaaa cgcacggtgt tgggtcgttt gttcataaac 3660
   gcggggttcg gtcccagggc tggcactctg tcgatacccc accgagaccc cattggggcc 3720
   aatacgcccg cgtttcttcc ttttccccac cccacccccc aagttcgggt gaaggcccag 3780
   ggctcgcagc caacgtcggg gcggcaggcc ctgccatagc ctcaggttac tcatatatac 3840
   tttagattga tttaaaactt catttttaat ttaaaaggat ctaggtgaag atcctttttg 3900
   ataatctcat gaccaaaatc ccttaacgtg agttttcgtt ccactgagcg tcagaccccg    3960
   tagaaaagat caaaggatct tcttgagatc ctttttttct gcgcgtaatc tgctgcttgc    4020
   aaacaaaaaa accaccgcta ccagcggtgg tttgtttgcc ggatcaagag ctaccaactc    4080
   tttttccgaa ggtaactggc ttcagcagag cgcagatacc aaatactgtc cttctagtgt    4140
   agccgtagtt aggccaccac ttcaagaact ctgtagcacc gcctacatac ctcgctctgc    4200
   taatcctgtt accagtggct gctgccagtg gcgataagtc gtgtcttacc gggttggact    4260
   caagacgata gttaccggat aaggcgcagc ggtcgggctg aacggggggt tcgtgcacac    4320
   agcccagctt ggagcgaacg acctacaccg aactgagata cctacagcgt gagctatgag    4380
   aaagcgccac gcttcccgaa gggagaaagg cggacaggta tccggtaagc ggcagggtcg    4440
   gaacaggaga gcgcacgagg gagcttccag ggggaaacgc ctggtatctt tatagtcctg    4500
   tcgggtttcg ccacctctga cttgagcgtc gatttttgtg atgctcgtca ggggggcgga    4560
   gcctatggaa aaacgccagc aacgcggcct ttttacggtt cctggccttt tgctggcctt    4620
   ttgctcacat gttctttcct gcgttatccc ctgattctgt ggataaccgt attaccgcca    4680
   tgcat                                                                4685
<210> 23
   <211> 32
   <212> DNA
   <213> Artificial Sequence
<220>
   <223> PRKCD-F
<400> 23
   gaagctagcc gccaccatgg cgccgttcct gc 32
<210> 24
   <211> 37
   <212> DNA
   <213> Artificial Sequence
<220>
   <223> PRKCD-R
<400> 24
   gaaaccggtg gatcttccag gaggtgctcg aatttgg 37
<210> 25
   <211> 43
   <212> DNA
   <213> Artificial Sequence
<220>
   <223> TMA-F
<400> 25
   gaagctagcc gccaccatga aacaggccaa aatccactac atc 43
<210> 26
   <211> 32
   <212> DNA
   <213> Artificial Sequence
<220>
   <223> TMA-R
<400> 26
   gaaaccggtg gagtgtcccg gctgttggcc gc 32
<210> 27
   <211> 41
   <212> DNA
   <213> Artificial Sequence
<220>
   <223> TMB-F
<400> 27
   gcagctagcc gccaccatgc agaaagaacg cttcaacatc g 41
<210> 28
   <211> 32
   <212> DNA
   <213> Artificial Sequence
<220>
   <223> TMB-R
<400> 28
   gcaaccggtg gggcctcagc caaaagcttc tg 32
<210> 29
   <211> 32
   <212> DNA
   <213> Artificial Sequence
<220>
   <223> PKCD-F
<400> 29
   gaagctagcc gccaccatgg cgccgttcct gc 32
<210> 30
   <211> 21
   <212> DNA
   <213> Artificial Sequence
<220>
   <223> Y313F-R
<400> 30
   gaaaccctga aatatcccaa c 21
<210> 31
   <211> 21
   <212> DNA
   <213> Artificial Sequence
<220>
   <223> Y313F-F
<400> 31
   gttgggatat ttcagggttt c 21
<210> 32
   <211> 37
   <212> DNA
   <213> Artificial Sequence
<220>
   <223> PKCD-R
<400> 32
   gaaaccggtg gatcttccag gaggtgctcg aatttgg 37
<210> 33
   <211> 21
   <212> DNA
   <213> Artificial Sequence
<220>
   <223> K378R-F
<400> 33
   tttgccatca gggccctcaa g 21
<210> 34
   <211> 21
   <212> DNA
   <213> Artificial Sequence
<220>
   <223> K378R-R
<400> 34
   cttgagggcc ctgatggcaa a 21
<210> 35
   <211> 44
   <212> DNA
   <213> Artificial Sequence
<220>
   <223> NLS-F-1
<400> 35
   agtaaaaagg aaaaggataa atagataact gatcataatc agcc 44
<210> 36
   <211> 23
   <212> DNA
   <213> Artificial Sequence
<220>
   <223> NLS-R
<400> 36
   gctgcaataa acaagttaac aac 23
<210> 37
   <211> 44
   <212> DNA
   <213> Artificial Sequence
<220>
   <223> NLS-F-2
<400> 37
   tggaagaagt agctaagaag aagagtaaaa aggaaaagga taaa 44
<210> 38
   <211> 44
   <212> DNA
   <213> Artificial Sequence
<220>
   <223> NLS-F-3
<400> 38
   tccggtgatg aagtcgaagg agtggaagaa gtagctaaga agaa 44
<210> 39
   <211> 33
   <212> DNA
   <213> Artificial Sequence
<220>
   <223> NLS-F-4
<400> 39
   gctggatcca ggctctggtg atgaagtcga agg 33
<210> 40
   <211> 44
   <212> DNA
   <213> Artificial Sequence
<220>
   <223> NES-F-1
<400> 40
   gtgggaaaca tttccctggt gtagataact gatcataatc agcc 44
<210> 41
   <211> 23
   <212> DNA
   <213> Artificial Sequence
<220>
   <223> NES-R
<400> 41
   gctgcaataa acaagttaac aac 23
<210> 42
   <211> 44
   <212> DNA
   <213> Artificial Sequence
<220>
   <223> NES-F-2
<400> 42
   gtcatcatca agctgaacgc ccatgtggga aacatttccc tggt 44
<210> 43
   <211> 40
   <212> DNA
   <213> Artificial Sequence
<220>
   <223> NES-F-3
<400> 43
   gtcggatcca gaccagcgcg tcatcatcaa gctgaacgcc 40
<210> 44
   <211> 39
   <212> DNA
   <213> Artificial Sequence
<220>
   <223> AzG-F
<400> 44
   ggcaccggtc gccaccatgg accccatggt gagtgtgat 39
<210> 45
   <211> 35
   <212> DNA
   <213> Artificial Sequence
<220>
   <223> AzG-R
<400> 45
   ggcagatctg acagcttggc ctgactcggc agcat 35
<210> 46
   <211> 24
   <212> DNA
   <213> Artificial Sequence
<220>
   <223> HcR-F
<400> 46
   gccaccggtc gccaccatgg tgag 24
<210> 47
   <211> 38
   <212> DNA
   <213> Artificial Sequence
<220>
   <223> HcR-R
<400> 47
   gccgcggccg cttatcagtt ggccttctcg ggcaggtc 38
<210> 48
   <211> 35
   <212> DNA
   <213> Artificial Sequence
<220>
   <223> p53-F
<400> 48
   gaagaattct gatgcctgtc accgagaccc ctggg 35
<210> 49
   <211> 34
   <212> DNA
   <213> Artificial Sequence
<220>
   <223> p53-R
<400> 49
   gaaggatccc gtcagtctga gtcaggcccc actt 34
<210> 50
   <211> 35
   <212> DNA
   <213> Artificial Sequence
<220>
   <223> SV40T-F
<400> 50
   gaagaattct gatgggaact gatgaatggg agcag 35
<210> 51
   <211> 32
   <212> DNA
   <213> Artificial Sequence
<220>
   <223> SV40T-R
<400> 51
   gaaggatccc gttatgtttc aggttcaggg gg 32
<210> 52
   <211> 30
   <212> DNA
   <213> Artificial Sequence
<220>
   <223> p38-F
<400> 52
   gtcctcgaga tgccgatgta ccaggtaaag 30
<210> 53
   <211> 30
   <212> DNA
   <213> Artificial Sequence
<220>
   <223> p38-R
<400> 53
   gtcggatcct taaaaaggag ccaggttttc 30
<210> 54
   <211> 32
   <212> DNA
   <213> Artificial Sequence
<220>
   <223> Gag-F
<400> 54
   gtcgaattct gatgggtgcg agagcgtcag ta 32
<210> 55
   <211> 30
   <212> DNA
   <213> Artificial Sequence
<220>
   <223> Gag-R
<400> 55
   gtcggatcct tattgtgacg aggggtcgtt 30
<210> 56
   <211> 31
   <212> DNA
   <213> Artificial Sequence
<220>
   <223> LR-F
<400> 56
   gtcgaattct gatgtccgga gcccttgatg t 31
<210> 57
   <211> 32
   <212> DNA
   <213> Artificial Sequence
<220>
   <223> LR-R
<400> 57
   gtcggatcct taagaccagt cagtggttgc tc 32
<210> 58
   <211> 29
   <212> DNA
   <213> Artificial Sequence
<220>
   <223> KRS-F
<400> 58
   gtcgaattct gatggcggcc gtgcaggcg 29
<210> 59
   <211> 34
   <212> DNA
   <213> Artificial Sequence
<220>
   <223> KRS-R
<400> 59
   gtccccgggc tagacagaag tgccaactgt tgtg 34
<210> 60
   <211> 30
   <212> DNA
   <213> Artificial Sequence
<220>
   <223> RelA-F
<400> 60
   ggactcgaga tggacgaact gttccccctc 30
<210> 61
   <211> 36
   <212> DNA
   <213> Artificial Sequence
<220>
   <223> RelA-R
<400> 61
   gaaggatccc gttaggagct gatctgactc agcagg 36
<210> 62
   <211> 31
   <212> DNA
   <213> Artificial Sequence
<220>
   <223> IkB-F
<400> 62
   gaagaattct gatgttccag gcggccgagc g 31
<210> 63
   <211> 38
   <212> DNA
   <213> Artificial Sequence
<220>
   <223> IkB-R
<400> 63
   gaaggatccc gtcataaacg tcagacgctg gcctccaa 38
<210> 64
   <211> 33
   <212> DNA
   <213> Artificial Sequence
<220>
   <223> p50-F
<400> 64
   gctgaattct gatggcagaa gatgatccat att 33
<210> 65
   <211> 34
   <212> DNA
   <213> Artificial Sequence
<220>
   <223> p50-R
<400> 65
   gctcccgggc ttaatgcttc atcccagcat taga 34
<210> 66
   <211> 33
   <212> DNA
   <213> Artificial Sequence
<220>
   <223> OmpA-F
<400> 66
   gctgaattct gatgaaattg agtcgtattg cac 33
<210> 67
   <211> 30
   <212> DNA
   <213> Artificial Sequence
<220>
   <223> OmpA-R
<400> 67
   gctggatcct tattgagctg ctgcaggagc 30
<210> 68
   <211> 31
   <212> DNA
   <213> Artificial Sequence
<220>
   <223> EEF1A-F
<400> 68
   gctgaattct gatgggaaag gaaaagactc a 31
<210> 69
   <211> 31
   <212> DNA
   <213> Artificial Sequence
<220>
   <223> EEF1A-R
<400> 69
   gctggatccc gctatttagc cttctgagct t 31
<210> 70
   <211> 32
   <212> DNA
   <213> Artificial Sequence
<220>
   <223> FAM1 4B-F
<400> 70
   gtcgaattct gatgggaaag gagagtggat gg 32
<210> 71
   <211> 31
   <212> DNA
   <213> Artificial Sequence
<220>
   <223> FAM1 4B-R
<400> 71
   gtcggatccc gtcagctgga agggggtgaa c 31
<210> 72
   <211> 32
   <212> DNA
   <213> Artificial Sequence
<220>
   <223> DDX31-F
<400> 72
   gtcgaattct gatgttttct ccaaagaagc at 32
<210> 73
   <211> 33
   <212> DNA
   <213> Artificial Sequence
<220>
   <223> DDX31-R
<400> 73
   gtcggatccc gttaaacttt ctgggaagtc ttg 33
<210> 74
   <211> 32
   <212> DNA
   <213> Artificial Sequence
<220>
   <223> p53N-F
<400> 74
   gtcgaattct catggaggag ccgcagtcag at 32
<210> 75
   <211> 29
   <212> DNA
   <213> Artificial Sequence
<220>
   <223> p53N-R
<400> 75
   gtcggatcct cacacggggg gagcagcct 29
<210> 76
   <211> 37
   <212> DNA
   <213> Artificial Sequence
<220>
   <223> mdm2N-F
<400> 76
   gtcgaattct gatgtgcaat accaacatgt ctgtacc 37
<210> 77
   <211> 33
   <212> DNA
   <213> Artificial Sequence
<220>
   <223> mdm2N-R
<400> 77
   gtcggatcct catactacca agttcctgta gat 33
<210> 78
   <211> 13
   <212> PRT
   <213> Homo sapiens
<220>
   <221> PEPTIDE
   <222> (1) .. (11)
   <223> Annexin II
<400> 78
   Val His Glu Ile Leu Cys Lys Xaa Xaa Leu Ser Leu Glu
    1               5                   10
<210> 79
   <211> 15
   <212> PRT
   <213> Homo sapiens
<220>
   <221> PEPTIDE
   <222> (1) .. (15)
   <223> mNet
<400> 79
   Thr Leu Trp Gln Phe Xaa Leu Leu His Xaa Xaa Leu Leu Leu Asp
    1               5                   10                  15
<210> 80
   <211> 15
   <212> PRT
   <213> Homo sapiens
<220>
   <221> PEPTIDE
   <222> (1) .. (15)
   <223> hNet
<400> 80
   Thr Leu Trp Gln Phe Xaa Leu Leu Gln Xaa Xaa Leu Leu Leu Asp
    1               5                   10                  15
<210> 81
   <211> 15
   <212> PRT
   <213> Homo sapiens
<220>
   <221> PEPTIDE
   <222> (1) .. (15)
   <223> MAPKK
<400> 81
   Ala Leu Gln Lys Lys Xaa Leu Glu Glu Xaa Xaa Leu Glu Leu Asp
    1               5                   10                  15
<210> 82
   <211> 15
   <212> PRT
   <213> Homo sapiens
<220>
   <221> PEPTIDE
   <222> (1)..(15)
   <223> PKI
<400> 82
   Glu Leu Ala Leu Lys Xaa Leu Ala Gly Xaa Xaa Leu Asp Ile Asn
   1               5                   10                  15
<210> 83
   <211> 14
   <212> PRT
   <213> Homo sapiens
<220>
   <221> PEPTIDE
   <222> (1)..(14)
   <223> Rev
<400> 83
   Leu Gln Leu Pro Pro Leu Glu Arg Xaa Xaa Leu Thr Leu Asp
   1               5                   10
<210> 84
   <211> 13
   <212> PRT
   <213> Homo sapiens
<220>
   <221> PEPTIDE
   <222> (1) .. (13)
   <223> Dsk-1
<400> 84
   Ser Leu Glu Gly Ala Val Ser Glu Ile Ser Xaa Leu Arg
   1               5                   10
<210> 85
   <211> 13
   <212> PRT
   <213> Homo sapiens
<220>
   <221> PEPTIDE
   <222> (1)..(13)
   <223> Cyclin B1
<400> 85
   Tyr Leu Cys Gln Ala Phe Ser Asp Val Ile Xaa Leu Ala
   1 5 10
<210> 86
   <211> 15
   <212> PRT
   <213> Homo sapiens
<220>
   <221> PEPTIDE
   <222> (1) .. (15)
   <223> ANXII
<400> 86
   Ser Thr Val His Glu Ile Leu Cys Lys Xaa Xaa Leu Ser Leu Glu
   1 5 10 15
<210> 87
   <211> 14
   <212> PRT
   <213> Homo sapiens
<220>
   <221> PEPTIDE
   <222> (1)..(14)
   <223> HIV-1 Rev
<400> 87
   Leu Gln Leu Pro Pro Leu Glu Arg Xaa Xaa Leu Thr Leu Asp
   1 5 10
<210> 88
   <211> 15
   <212> PRT
   <213> Homo sapiens
<220>
   <221> PEPTIDE
   <222> (1)..(15)
   <223> MEK-1
<400> 88
   Ala Leu Gln Lys Lys Xaa Leu Glu Glu Xaa Xaa Leu Glu Leu Asp
   1 5 10 15
<210> 89
   <211> 15
   <212> PRT
   <213> Homo sapiens
<220>
   <221> PEPTIDE
   <222> (1)..(15)
   <223> PKI-alpha
<400> 89
   Glu Leu Ala Leu Lys Xaa Leu Ala Gly Xaa Xaa Leu Asp Ile Asn
    1               5                  10                  15
<210> 90
   <211> 13
   <212> PRT
   <213> Homo sapiens
<220>
   <221> PEPTIDE
   <222> (1)..(13)
   <223> IkB-alpha
<400> 90
   Ile Gln Gln Gln Leu Gly Gln Xaa Xaa Leu Thr Leu Glu
    1               5                  10
<210> 91
   <211> 14
   <212> PRT
   <213> Homo sapiens
<220>
   <221> PEPTIDE
   <222> (1)..(14)
   <223> RanBP1
<400> 91
   Lys Asx Ala Glu Lys Leu Glu Ala Xaa Xaa Leu Ser Val Arg
    1               5                  10
<210> 92
   <211> 18
   <212> PRT
   <213> Homo sapiens
<220>
   <221> PEPTIDE
   <222> (1)..(18)
   <223> INI1
<400> 92
   Asp Gln Arg Val Ile Ile Lys Leu Asn Ala His Val Gly Asn Ile Ser
   1 5 10 15
   Leu Val
<210> 93
   <211> 7
   <212> PRT
   <213> Simian virus 40
<220>
   <221> PEPTIDE
   <222> (1) .. (7)
   <223> SV40 T Antigen
<400> 93
   Pro Lys Lys Lys Arg Lys Val
   1 5
<210> 94
   <211> 7
   <212> PRT
   <213> Saccharomyces cerevisiae
<220>
   <221> PEPTIDE
   <222> (1)..(7)
   <223> Yeast histone H2B
<400> 94
   Gly Lys Lys Arg Ser Lys Val
   1 5
<210> 95
   <211> 9
   <212> PRT
   <213> Homo sapiens
<220>
   <221> PEPTIDE
   <222> (1)..(9)
   <223> Human c-myc
<400> 95
   Pro Ala Ala Lys Arg Val Lys Leu Asp
   1 5
<210> 96
   <211> 17
   <212> PRT
   <213> Homo sapiens
<220>
   <221> PEPTIDE
   <222> (1)..(17)
   <223> Nucleoplasmin
<400> 96
   Lys Arg Pro Ala Ala Thr Lys Lys Ala Gly Gln Ala Lys Lys Lys Lys
   1               5                   10                  15
   Leu
<210> 97
   <211> 20
   <212> PRT
   <213> Homo sapiens
<220>
   <221> PEPTIDE
   <222> (1) .. (20)
   <223> Human IL-5
<400> 97
   Lys Lys Tyr Thr Asp Gly Gln Lys Lys Lys Cys Gly Glu Glu Arg Arg
   1               5                   10                  15
   Arg Val Asn Gln
               20
<210> 98
   <211> 17
   <212> PRT
   <213> Homo sapiens
<220>
   <221> PEPTIDE
   <222> (1)..(17)
   <223> Human RB
<400> 98
   Lys Arg Ser Ala Glu Gly Ser Asn Pro Pro Lys Pro Leu Lys Lys Leu
   1               5                   10                  15
   Arg
<210> 99
   <211> 18
   <212> PRT
   <213> Homo sapiens
<220>
   <221> PEPTIDE
   <222> (1) .. (18)
   <223> Human p53
<400> 99
   Lys Arg Ala Leu Pro Asn Asn Thr Ser Ser Ser Pro Gln Pro Lys Lys
    1               5                  10                  15
   Lys Pro